(19) Europäisches Patentamt / European Patent Office / Office européen des brevets

(11) **EP 2 687 151 A1**

(12) **EUROPEAN PATENT APPLICATION**

(43) Date of publication:
**22.01.2014 Bulletin 2014/04**

(51) Int Cl.:
***A61B 5/00*** (2006.01)

(21) Application number: **13003670.0**

(22) Date of filing: **22.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.07.2012 JP 2012161478**
**16.07.2013 JP 2013147258**

(71) Applicants:
• **Tanita Corporation**
**Tokyo 174-8630 (JP)**

• **National University Corporation Tokyo University of Agriculture and Technology**
**Fuchu-shi, Tokyo 183-8538 (JP)**

(72) Inventors:
• **Inoue, Kazuma**
**Tokyo, 174-8630 (JP)**
• **Sakuma, Atsushi**
**Fuchu-shi Tokyo, 183-8538 (JP)**

(74) Representative: **Müller-Boré & Partner**
**Patentanwälte PartG mbB**
**Grafinger Straße 2**
**81671 München (DE)**

(54) **Viscoelasticity measuring apparatus**

(57) A viscoelasticity measuring apparatus that measures viscoelasticity of a measurement target with high precision is provided. The measuring apparatus includes: a casing; a surface contact part provided in the casing and brought into surface contact with skin; a ball indenter that moves toward the skin more than the surface contact part and is pushed into the skin; a driving unit that supports the ball indenter and moves the ball indenter toward the skin; a load cell whose right end side is fixed to the casing and left end side supports the driving unit, the load cell detecting a pushing load that pushes the ball indenter into the skin; and a control unit that obtains displacement of the ball indenter.

FIG. 2A

# FIG. 2B

**Description**

This application is based on and claims the benefit of priority from Japanese Patent Application No. 2012-161478, filed on 20 July 2012, and Japanese Patent Application No. 2013-147258, filed on 16 July 2013,the contents of which are incorporated herein by reference.

BACKGROUND OF THE INVENTION

Field of the Invention

The present invention relates to a viscoelasticity measuring apparatus that measures viscoelasticity of a measurement target.

Related Art

A measuring apparatus for measuring viscoelasticity of a measurement target such as human skin has been known heretofore (for example, Japanese Unexamined Patent Application Publication No. 2000-316818, hereinafter referred to as Patent Document 1). In the invention of Patent Document 1, a pressure sensor unit provided with a probe measures a pressure applied to the measurement target by driving a driving unit.

However, in the invention of Patent Document 1, since the driving unit drives the probe and the pressure sensor unit integrally, the driving unit structure is large and its weight  is heavy. As a result, the influence of shaking of a measurer's hand holding the measuring apparatus on measuring precision of the pressure sensor becomes greater. In addition, the driving force of an actuator must be greater for the increased weight.

Furthermore, in the invention of Patent Document 1, the distance between the actuator and the probe is large due to the pressure sensor interposed therebetween. This increases axial runout of the actuator and the influence on measuring precision. Additional components such as bearing and coupling for controlling the shaking are thus required, leading to increased cost. The bearing generates slide friction and has an influence on measuring precision especially when handling low pressure.

Moreover, in the invention of Patent Document 1, a spring presses the pressure sensor to prevent shaking and the apparatus is fixed to a ball screw device, so that the sensor fixing state is unstable due to backlash and the like. In order to perform sensing of higher precision, the fixing state of the pressure sensor must be more stable.

For those reasons, the invention of Patent Document 1 is not suitable for high precision measurement that handles minute pressure.

SUMMARY OF THE INVENTION

A problem to be solved by the present invention is to provide a viscoelasticity measuring apparatus that measures viscoelasticity of a measurement target with high precision.

The present invention solves the problem by the following means.

According to a first aspect of the present invention, there is provided a viscoelasticity measuring apparatus comprising a casing; a surface contact part provided in the casing and brought into surface contact with a measurement target; an indenter that moves toward the measurement target over the surface contact part and is pushed into the measurement target; a driving unit that supports the indenter and moves the indenter toward the measurement target; a load detecting unit of which a fixed part side is fixed to the casing and a movable part side supports the driving unit, wherein the load detecting unit detects a pushing load that pushes the indenter into the measurement target; and a displacement obtaining unit that obtains displacement of the indenter.

The above described viscoelasticity measuring apparatus may comprise a control unit, wherein the control unit performs: an indenter pushing process in which the control unit controls the driving unit to position the indenter at a pushing position; and a viscoelasticity calculating process in which the control unit calculate viscoelasticity of the measurement target at the pushing position of the indenter pushing process, based on output from the load detecting unit and the displacement of the indenter obtained by the displacement obtaining unit.

In the above described viscoelasticity measuring apparatus, the driving unit may include a pulse motor; and the control unit may control the pulse motor by outputting a driving pulse, obtains displacement of the indenter based on the driving pulse, and functions as the displacement obtaining unit.

In the above described viscoelasticity measuring apparatus, the control unit may perform the viscoelasticity calculating process by adding displacement of the load detecting unit as displacement of the indenter.

The above described viscoelasticity measuring apparatus may further comprise a contact angle correcting unit that corrects a contact angle of the indenter with respect to a surface of the measurement target to be orthogonal to the surface of the measurement target.

The above described viscoelasticity measuring apparatus may further comprise a contact pressure correcting unit that corrects appropriately pressure of contact between the surface contact part and the measurement target.

In the above described viscoelasticity measuring apparatus, the viscoelasticity measuring apparatus may be used in such a way that a measurer holds the casing and brings the surface contact part into contact with the measurement target, and may further comprise a vibration reducing unit that reduces vibration between the surface contact part and the measurement target.

According to the present invention, a viscoelasticity measuring apparatus that measures viscoelasticity of a measurement target with high precision can be provided.

The above described viscoelasticity measuring apparatus may comprise: an inclination detecting unit for detecting inclination of a detecting direction of the load detecting unit; and a control unit, wherein the control unit corrects a detection reference point of the load detecting unit by correcting the amount corresponding to the inclination of the detecting direction detected by the inclination detecting unit with respect to the output of load detecting unit.

In the above described viscoelasticity measuring apparatus, the inclination detecting unit may detect the detecting direction by detecting acceleration; and the control unit may correct the detection reference point of the load detecting unit by offsetting the amount corresponding to the acceleration detected by the inclination detecting unit with respect to the output of the load detecting unit.

The above described viscoelasticity measuring apparatus may comprise a pressing force detecting unit for detecting pressing force on the surface contact part; and a control unit, wherein the control unit determines whether the viscoelasticity is measured or not, based on the output of the pressing force detecting unit.

In the above described viscoelasticity measuring apparatus, the control unit may measure the viscoelasticity when the control unit has determined output of the pressing force on the surface contact part is within a predetermined pressure on the basis of the pressing force detecting unit.

The above described viscoelasticity measuring apparatus may comprise a plurality of the pressing force detecting units for detecting the pressing force at a plurality of positions on the surface contact part, wherein the control unit may measure the viscoelasticity when the control unit has determined that a relative value of the pressing force at the positions is within a predetermined range on the basis of the output of the pressing force detecting units.

In the above described viscoelasticity measuring apparatus, the control unit may correct the detection reference point of the pressing force detecting unit on the basis of the output of the inclination detecting unit.

The above described viscoelasticity measuring apparatus may comprise: a plurality of the pressing force detecting units for detecting the pressing force at a plurality of positions on the surface contact part; and a pressing force display unit for displaying the pressing force, wherein the control unit allows the pressing force display unit to display a central position of operation of the pressing force among the positions on the basis of the output of the pressing force detecting units.

The above described viscoelasticity measuring apparatus may comprise a pressing force display unit for displaying the pressing force, wherein the control unit allows the pressing force display unit to display the pressing force in a mode of being able to identify the level of the pressing force on the basis of the output of the pressing force detecting unit.

The above described viscoelasticity measuring apparatus may comprise: a pressing force detecting unit for detecting pressing force on the surface contact part; and a control unit, wherein the control unit calculates the viscoelasticity of the measurement target at a plurality of levels of pressing force with different output of the pressing force detecting unit, and obtains the viscoelasticity corresponding to a lower level of pressing force than the plurality of levels of pressing force on the basis of the calculated plurality of viscoelasticity.

BRIEF DESCRIPTION OF THE DRAWINGS

FIG. 1A is a perspective view of a measuring apparatus of a first embodiment;
FIG. 1B is a figure illustrating a usage situation of the measuring apparatus of the first embodiment;
FIG. 2A is a top view of the measuring apparatus of the first embodiment;
FIG. 2B is a cross-sectional view of the measuring apparatus of the first embodiment;
FIG. 3A is an enlarged cross-sectional view of illustrating a vicinity of a driving unit (a part indicated by an arrow III in FIG. 2B) in the first embodiment, in a state in which a ball indenter is positioned at an evacuated position;
FIG. 3B is an enlarged cross-sectional view illustrating a vicinity of a driving unit (part indicated by an arrow III in FIG. 2B) in the first embodiment, in a state in which the ball indenter is projected;
FIG. 4 is a block diagram of the measuring apparatus of the first embodiment;
FIG. 5 is a flow chart of processing of the measuring apparatus of the first embodiment;

FIG. 6A is a top view of the measuring apparatus of a second embodiment;

FIG. 6B is a cross-sectional view of the measuring apparatus of the second embodiment (corresponding to FIG. 2);

FIG. 7A is a perspective view of a measuring apparatus of a third embodiment;

FIG. 7B is a perspective view of the measuring apparatus of the third embodiment;

FIG. 7C is a perspective view of the measuring apparatus of the third embodiment;

FIG. 8A is a perspective view of a measuring apparatus of the third embodiment;

FIG. 8B is a cross-sectional view of the measuring apparatus of the third embodiment;

FIG. 8C is another perspective view of the measuring apparatus of the third embodiment;

FIG. 8D is another cross-sectional view of the measuring apparatus of the third embodiment;

FIG. 9A is one of a three-view drawing of the measuring apparatus of the third embodiment;

FIG. 9B is one of a three-view drawing of the measuring apparatus of the third embodiment;

FIG. 9C is one of a three-view drawing of the measuring apparatus of the third embodiment;

FIG. 10A is one of a three-view drawing of the measuring apparatus of the third embodiment;

FIG. 10B is one of a three-view drawing of the measuring apparatus of the third embodiment;

FIG. 10C is one of a three-view drawing of the measuring apparatus of the third embodiment;

FIG. 11 is a perspective view of the measuring apparatus of the third embodiment;

FIG. 12A is a perspective view of a measuring apparatus 401 of a fourth embodiment;

FIG. 12B is a cross-sectional view of the measuring apparatus of the fourth embodiment;

FIG. 13 is a block diagram of the measuring apparatus of the fourth embodiment;

FIGS. 14A to 14C are graphs for describing the measurement error in the inclination of the measuring apparatus;

FIGS. 15A to 15D are graphs representing the output of a load cell and an acceleration sensor of the fourth embodiment;

FIGS. 16A and 16B are graphs representing the measurement results of the load and displacement of skin when a subject is in a shaking state;

FIGS. 17A and 17B are explanatory views illustrating inhomogeneous and homogenous states of the pressing force distribution of an annular part of the fourth embodiment;

FIG. 18 is a flow chart of a set of processes of the measuring apparatus of the fourth embodiment;

FIG. 19 illustrates a pressing force information display screen of the fourth embodiment; and

FIG. 20 illustrates the relation between the Young's modulus and the pressing force of the annular part.

## DETAILED DESCRIPTION OF THE INVENTION

(First Embodiment)

A first embodiment of the present invention is described hereinafter with reference to the drawings.

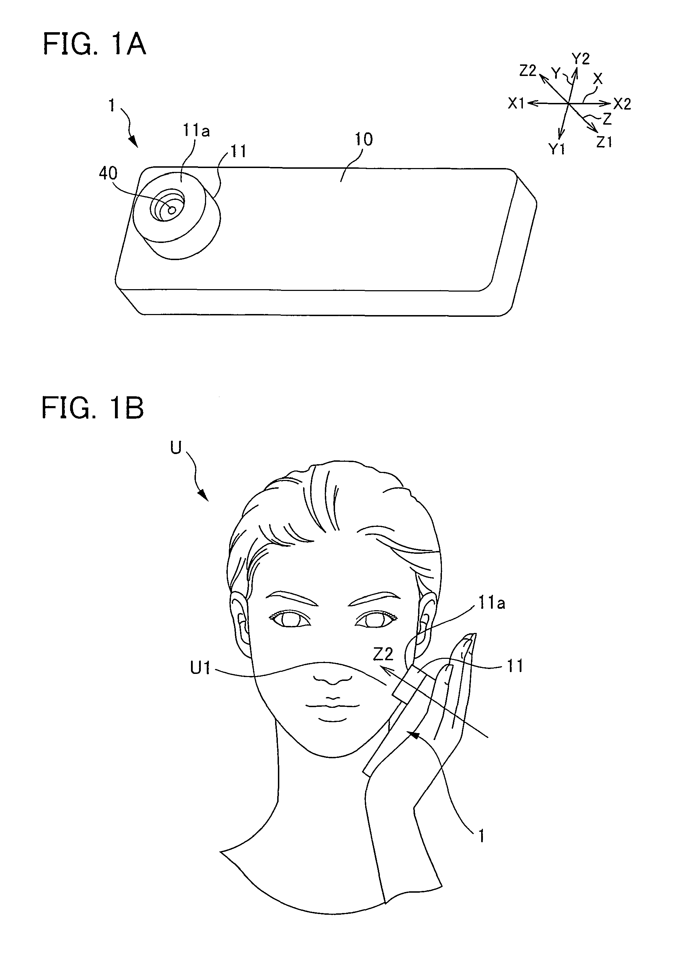

FIG. 1A is a perspective view of the measuring apparatus 1.

FIG. 1B is a figure illustrating a usage situation of the measuring apparatus 1.

In descriptions of the embodiment and drawings, right and left direction, up and down direction, and thickness direction in FIG. 1A are referred to as directions X, Y, and Z.

The measuring apparatus 1 is a cuboid that is elongated in the right and left direction X. The measuring apparatus 1 is sized to fit in the palm of one's hand. The measuring apparatus 1 is a viscoelasticity measuring apparatus that pushes a ball indenter 40 onto the skin U1 and measures viscoelasticity of the skin U1.

A measurer U, who is a user, places the measuring apparatus 1 in his/her palm and puts an annular part 11 against the skin U1 (measurement target) such as the cheek.

The measurer U can also grasp the measuring apparatus 1.

By reducing the size of the measuring apparatus 1, the measurer can perform the measurement by putting the measuring apparatus against the cheek while putting a part of his/her palm not used for holding the measuring apparatus 1, against a face. As a result, the influence of shaking of hand can thus be reduced.

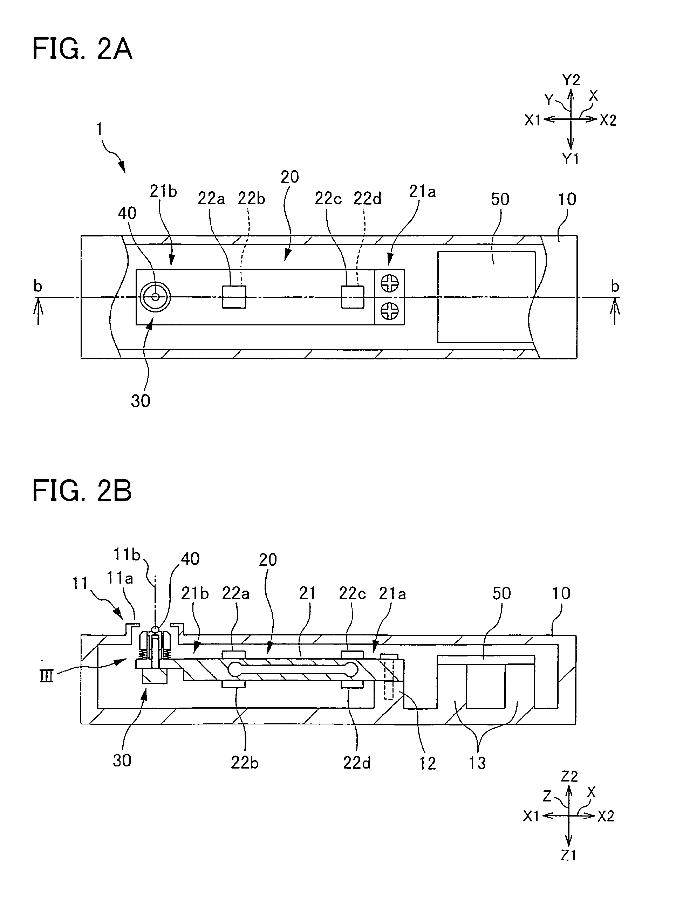

FIG. 2A is a top view illustrating an internal configuration of the measuring apparatus 1.

FIG. 2B is a cross-sectional view taken along the line b-b of FIG. 2A.

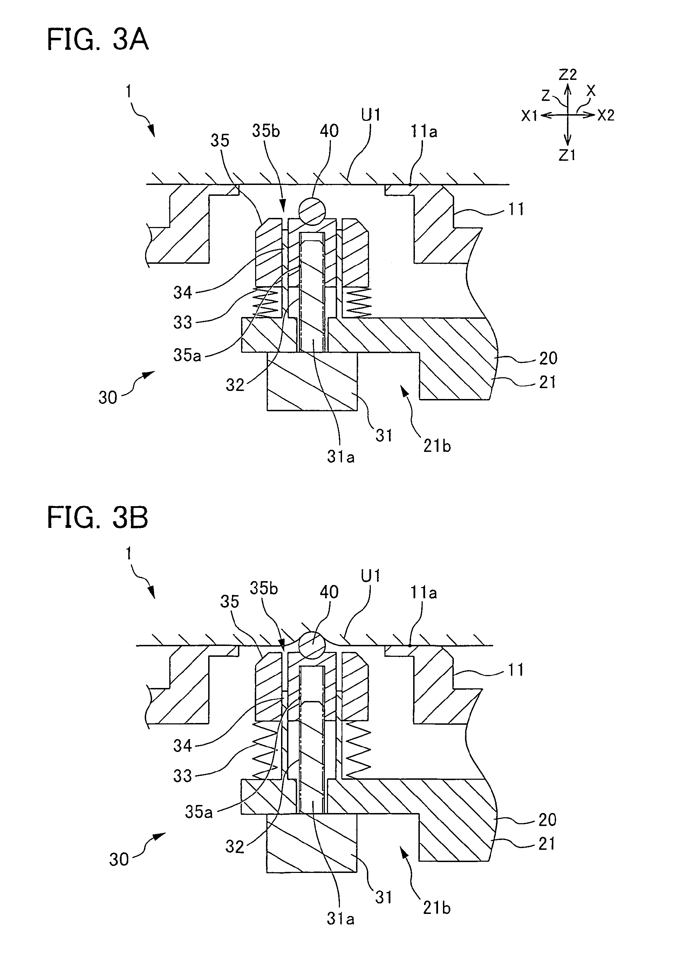

FIGS. 3A and 3B are enlarged cross-sectional views illustrating a vicinity of a driving unit 30 (a part indicated by the arrow III in FIG. 2B) in the first embodiment.

FIG. 3A illustrates a state in which the ball indenter 40 is positioned at an evacuated position.

FIG. 3B illustrates a state in which the ball indenter 40 is projected.

As shown in FIG. 2, the measuring apparatus 1 includes: a casing 10; the annular part 11; a load cell 20 (load detecting unit); the driving unit 30; the ball indenter 40 (indenter); and a substrate 50.

The casing 10 is a housing of the measuring apparatus 1. The casing 10 is a cuboid that is elongated in the right and left direction X.

The annular part 11 is an annular cylindrical body fixed on a top face of the casing 10. The surface of the annular part 11 is a surface contact part 11a that is brought into surface contact with the user's skin U1. A central shaft 11b of the annular part 11 is parallel to the thickness direction Z.

The load cell 20 detects a load (pressure) applied to the skin U1.

The load cell 20 is configured such that four strain gauges 22a to 22d are attached to an flexure body 21 of a parallel beam shape.

The flexure body 21 is arranged such that a longitudinal direction thereof corresponds to the right and left direction X. A right end side 21a (fixed part side) of the flexure body 21 is fixed by a screw to an attachment boss 12 of the casing 10. A left end side 21b (movable part side) of the flexure body 21 is a free end and supports the driving unit 30.

By the above-described configuration, the load cell 20 measures the load applied to the skin U1 by measuring a load applied in the thickness direction Z to the left end side 21b of the flexure body 21 via the driving unit 30.

The driving unit 30 is designed to support the ball indenter 40 and move the ball indenter 40 in a pushing direction Z2 (toward the measurement target). In the thickness direction Z, a direction toward the annular part 11 of the casing 10 is the pushing direction Z2 and a direction opposite thereto is a retraction direction Z1.

As shown in FIG. 3, the driving unit 30 includes: a pulse motor 31; a feed screw 32; a spring 33; a guide 34; and a moving body 35.

A main body of the pulse motor 31 is fixed to the left end side 21b of the flexure body 21. The axial direction of a rotational shaft 31a of the pulse motor 31 is the thickness direction Z.

The feed screw 32 is a male thread provided on a circumference of the rotational shaft 31a of the pulse motor 31. The feed screw 32 engages with a female screw 35a of the moving body 35. As the feed screw 32 is rotationally driven, the feed screw 32 moves the moving body 35 in the thickness direction Z.

The spring 33 is a helical extension spring that prevents instability caused by clearance gap(e.g. backlash) of the feed screw 32. A lower end of the spring 33 is fixed to the flexure body 21 and an upper end of the spring 33 is fixed to the moving body 35. As a result, the spring 33 pulls the moving body 35 in the retraction direction Z1 (toward the flexure body 21). By providing two springs 33, the moving body 35 can be pulled in an equilibrated manner.

The guide 34 is a shaft body that guides the moving body 35 in a direction parallel to the thickness direction Z. The axial direction of a shaft of the guide 34 is the thickness direction Z. A lower end of the guide 34 is fixed to the left end side 21b of the flexure body 21. The guide 34 is inserted into the guide hole 35b of the moving body 35. Two guides 34 guide the moving body 35 in an equilibrated manner while preventing rotation of the moving body 35 in an X-Y plane.

The moving body 35 is a driven part that is moved by the driving unit 30 in the thickness direction Z, in the above-described configuration. The moving body 35 is formed of resin or the like, and is light-weight.

The ball indenter 40 is a spherical body that is projected from the surface contact part 11a in the pushing direction Z2 and pushed into the skin U1. The ball indenter 40 is fixed to a top face of the moving body 35 and moves in the thickness direction Z along the central shaft 11b of the annular part 11, integrally with the moving body 35.

The ball indenter 40 is bring up from the evacuated position (position shown in FIG. 3A), at which the ball indenter 40 is most retracted with respect to the surface contact part 11a, in the pushing direction Z2, to a position projected from the surface contact part 11a (position shown in FIG. 3B). A movement stroke of the ball indenter 40 is several millimeters.

Here, upon contact of the surface contact part 11a with the skin U1, the skin U1 in a central portion of the surface contact part 11a rises in the Z1 direction, due to a pressure applied to the skin U1. The amount the skin rises depends on the softness of the skin U1 and the force of the measurer holding the measuring apparatus when pushing the measuring apparatus 1 against the skin. This causes variation in contact area between the ball indenter 40 and the skin U1 in relation to the stroke of movement of the ball indenter 40, possibly influencing measurement. For example, in a case in which the amount the skin rises is large, the ball indenter 40 (at the evacuated position shown in FIG. 3A) may already be in contact with the skin U1 when the measurement is begun. On the other hand, in a case in which the amount the skin rises is small, the contact area sufficient for measurement may not be secured.

Given this, a mechanism that moves the annular part 11 in the Z direction can be provided. As a result, the contact area between the ball indenter 40 and the skin U1 can thus be adjusted. Furthermore, for measurement with higher precision, it is preferable to provide a mechanism that can fix the position of the annular part 11 at an adjustment position.

Alternatively, a pressure adjustment unit may be provided, such as a mechanism that maintains pressure applied to the skin U1 within a predetermined range and a mechanism that measures pressure fluctuation. For example, by providing a spring at a position where pressure can be absorbed, and monitoring degree of compression of the spring, the appropriateness of the pressure can be determined; timing for starting the measurement can be realized; and an error massage can be generated in a case in which a large fluctuation is observed during measurement.

With the above-described configuration, the driving unit 30 is only required to drive the ball indenter 40, which is light in weight, in the thickness direction Z without driving the load cell 20, which is heavy in weight. This allows reduction in size and weight of the driving unit 30. The influence of shaking of the hand of the measurer U holding the measuring apparatus 1 during measurement can thus be reduced. In addition, since the driving unit 30 can function with a reduced driving force, driving electricity and power consumption can be reduced.

Furthermore, in the measuring apparatus 1, distance between the driving unit 30 and the skin U1, which is a measurement target, can be reduced. Since this can suppress enhancement of axial runout of the driving unit 30 due to shaking of the hand and the like, the need for components such as a bearing and coupling for controlling the axial runout can be eliminated, and cost reduction can be realized. Moreover, there is no influence of slide friction generated by the bearing and the like, and measuring precision can be improved.

The measuring apparatus 1 is configured such that the load cell 20 is firmly fixed to the attachment boss 12 of the casing 10 by a screw (fixing unit), and the driving unit 30 is provided on the load cell. In the invention of the present application, such a configuration can alleviate a problem being specific to a configuration in which a load sensor is provided on the driving unit as in Patent Document 1. The problems are, that is to say fluctuation of initial load and reduction in repetitive precision that is caused by unstable fixing of a sensor due to backlash and the like.

The fixing means for the fixing unit is not limited to a screw, and an adhesive and the like can also be used.

As shown in FIGS. 2A and 2B, the substrate 50 is a printed-wiring assembly in which a CPU (central processing unit), a semiconductor storage device, or the like, are embedded. The substrate 50 is fixed to the attachment boss 13 of the casing 10. The substrate 50 is electrically connected to electric components of the measuring apparatus 1 via an electric cable and the like (not illustrated). The substrate 50 is also connected to a battery (not illustrated) and supplies power to the electric components.

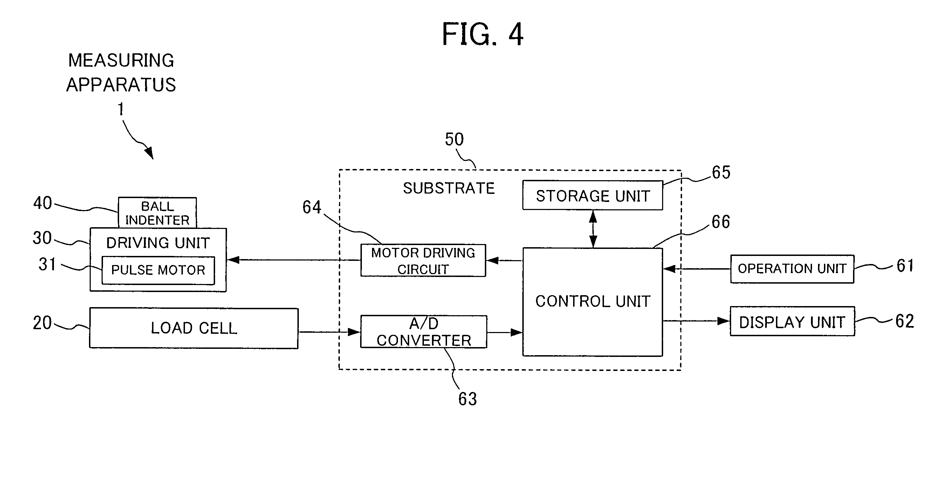

FIG. 4 is a block diagram of the measuring apparatus 1 of the first embodiment.

The measuring apparatus 1 includes: an operation unit 61; a display unit 62; an A/D converter 63; a motor driving circuit 64; a storage unit 65; and the control unit 66.

The operation unit 61 is provided to allow the measurer U to operate the measuring apparatus 1. The measuring apparatus 1 is provided with an operation button and the like (not illustrated in FIGS. 1 and 2) on the casing 10. The operation unit 61 outputs operation information to the control unit 66.

The display unit 62 is a display device that displays a measurement result. The display unit 62 is provided on the casing 10 (not illustrated in FIGS. 1 and 2).

The A/D converter 63 is an electric circuit that converts an analog signal from the load cell 20 into a digital signal and outputs to the control unit 66. The A/D converter 63 is embedded in the substrate 50.

The motor driving circuit 64 is a driving circuit of the pulse motor 31. The motor driving circuit 64 outputs voltage and the like suitable for driving of the pulse motor 31 based on a driving pulse output by the control unit 66. The motor driving circuit 64 is embedded in the substrate 50.

The storage unit 65 is a storage device for storing a program, information, and the like required for operation of the measuring apparatus 1. The storage unit 65 is composed of a storage device and the like installed on the substrate 50.

The control unit 66 is a control device that controls the measuring apparatus 1 in an overall manner. The control unit 66 is composed of a CPU and the like installed on the substrate 50.

The control unit 66 computes load of the load cell 20 based on an output from the A/D converter 63. The control unit 66 outputs a driving pulse to control the pulse motor 31 and counts the number of pulses of the driving pulse to obtain the displacement of the ball indenter 40. In other words, the control unit 66 also functions as the displacement obtaining unit that obtains displacement of the ball indenter 40.

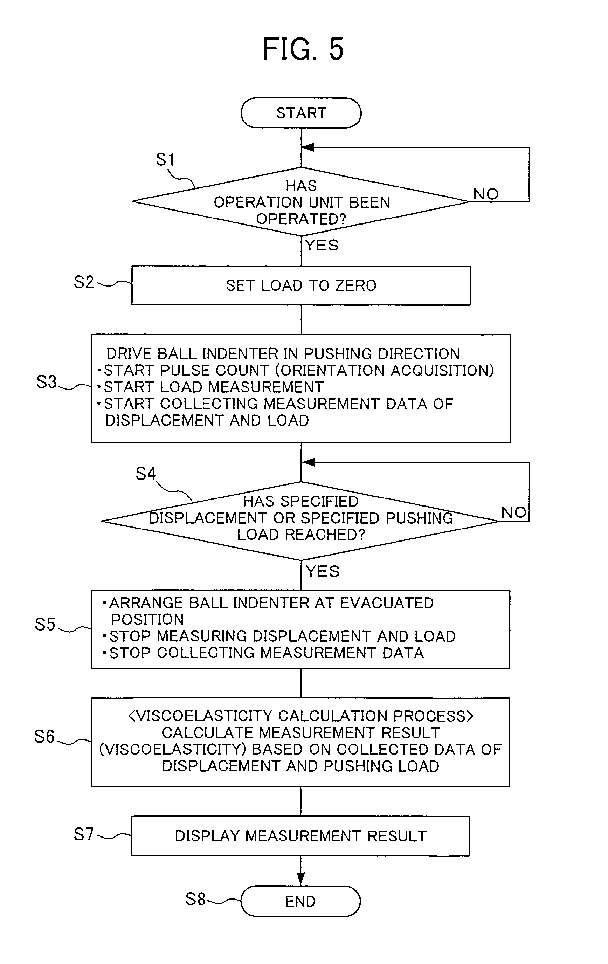

FIG. 5 is a flow chart of processing of the measuring apparatus 1 of the first embodiment.

Upon beginning measurement, the measurer U brings the surface contact part 11a in the annular part 11 of the measuring apparatus 1 into light contact with the skin U1. This state is shown in FIG. 3A. The measurer U operates the operation unit 61 in this state.

In step (hereinafter simply referred to as S) S1, the control unit 66 determines whether to start measurement or not, based on an output from the operation unit 61. In a case in which the control unit 66 determines to start measurement (S1: YES), the processing advances to S2. On the other hand, in a case in which the control unit 66 determines not to start measurement (S1: NO), the processing of S1 is repeated.

In S2, after the operation, the control unit 66 initializes (setting to zero) the load applied to the load cell 20 when the surface contact part 11a is put against the skin U1, which is the measuring target.

In S3, the control unit 66 outputs the driving pulse to drive the pulse motor 31. Upon starting driving of the pulse motor 31, the control unit 66 starts counting the driving pulses and obtains the displacement of the ball indenter 40 based on the number thus counted, and starts measuring the load by the load cell 20.

As shown in FIG. 3, the ball indenter 40 thus moves in the pushing direction Z2 and makes contact with the skin U1.

The control unit 66 continues to measure the displacement and pushing load at predetermined sampling intervals until reaching specified displacement or specified pushing load, and accumulates data.

In S4, the control unit 66 determines whether the pushing load has reached the specified pushing load or not, or whether the displacement of the ball indenter 40 has reached the specified displacement or not. In a case in which the control unit 66 determines that any of these conditions has been satisfied (S4: YES), the processing advances to S5. On the other hand, in a case in which the control unit 66 determines that none of these conditions has been satisfied (S4: NO), the processing of S4 is repeated. The processing of S4 is repeated until the pushing load reaches the specified pushing load or the displacement of the ball indenter 40 reaches the specified displacement (S4: YES).

In S5, the control unit 66 drives the ball indenter 40 in the retraction direction Z1 and positions at the evacuated position (see FIG. 3A). Meanwhile, the control unit 66 stops measurement of displacement and load, and terminates accumulation of data.

In S6, the control unit 66 performs a viscoelasticity calculating process. In this process, the control unit 66 calculates viscoelasticity of the skin U1 based on the accumulated data relating to displacement and load. A method for this calculation can be selected from among various calculation methods. For example, using Equations 28, 26 and 31 in Japanese Unexamined Patent Application Publication No. 2011-137667, non-linear physical properties of a three element solid model can be obtained: a Young's modulus of an elastic portion; viscosity compliance of a viscoelastic portion; and Young's modulus of an elastic portion in the viscoelastic portion. The equations corresponding to the above-cited equations 28, 26 and 31 respectively are as follows.

$$\text{Equation 28}: \quad E^e\left(\varepsilon_k^e\right) := \frac{\dot{\sigma}_{\alpha k}}{\dot{\varepsilon}_\alpha - C\left(\varepsilon_k^v\right)\left(\overline{\sigma}_{k+1} - \sigma_{\alpha k}^{ve}\right)}$$

$$\text{Equation 26}: \quad C\left(\varepsilon_k^v\right) := \frac{\dot{\sigma}_{\beta k}\dot{\varepsilon}_\alpha - \dot{\sigma}_{\alpha k}\dot{\varepsilon}_\beta + \left(1 - R_k\right)\dot{\sigma}_{\alpha k}\left(\overline{\sigma}_{k+1} - \sigma_{\beta k}^{ve}\right)C\left(\varepsilon_{k-1}^v\right)}{\dot{\sigma}_{\beta k}\left(\overline{\sigma}_{k+1} - \sigma_{\alpha k}^{ve}\right) - R_k\dot{\sigma}_{\alpha k}\left(\overline{\sigma}_{k+1} - \sigma_{\beta k}^{ve}\right)}$$

$$\text{Equation 31}: \quad E^{ve}\left(\varepsilon_k^v\right) := E^{ve}\left(\varepsilon_{k-1}^v\right) + \frac{\varepsilon_{\alpha k}^v - \varepsilon_{k-1}^v}{\varepsilon_{\gamma k}^v - \varepsilon_{k-1}^v}\left(\frac{E^e\left(\varepsilon_k^e\right)E_{\gamma k}}{E^e\left(\varepsilon_k^e\right) - E_{\gamma k}} - E^{ve}\left(\varepsilon_{k-1}^v\right)\right)$$

here:

$$R_k = \frac{\varepsilon_{\beta k}^v - \varepsilon_{k-1}^v}{\varepsilon_{\alpha k}^v - \varepsilon_{k-1}^v}$$

$E$ : Young's modulus
$C$ : viscosity compliance
$\varepsilon$ : strain
$\dot{\varepsilon}$ : strain rate
$\sigma$ : stress
$\dot{\sigma}$ : stress rate

The indices e, v, and ve respectively represent the elastic portion, the viscoelastic portion, and the elastic portion in the viscoelastic portion.

$$\dot{\varepsilon}_\alpha(\varepsilon_\alpha\,dot)\,、\quad \dot{\varepsilon}_\beta(\varepsilon_\beta\,dot)\,、\quad \dot{\varepsilon}_\gamma(\varepsilon_\gamma\,dot)$$

The above equation is a strain rate with a relationship of ($\varepsilon\alpha$ dot) > ($\varepsilon\beta$ dot) > ($\varepsilon\gamma$ dot), in which ($\varepsilon\alpha$ dot) and ($\varepsilon\beta$ dot) represent relatively high strain rates and ($\varepsilon\gamma$ dot) represents a strain rate extremely low compared to the ($\varepsilon\alpha$ dot) and ($\varepsilon\beta$ dot).

In addition, in the embodiments, the measurement target is the skin U1, which is thin. Given this, using Equations 31 and 34 in PCT International Application Publication No WO2010/084840 that are suitable for a thin measurement target, Young's modulus E was obtained as elasticity of the skin U1.

The components in the present embodiment corresponding to the above-cited equations 31 and 34, respectively, are as follows.

$$\text{Equation 31}:\quad \hat{F}=\frac{4}{3}\frac{E}{1-\nu^2}\left(\frac{\phi}{2}\right)^{\frac{1}{2}}\{\delta(1+B\delta)\}^{\frac{3}{2}}$$

$$\text{Equation 34}:\quad E=-\frac{6}{\pi^3\left(1-\nu^2\right)^2}\left(\frac{2}{\phi}\right)^2\frac{\hat{F}}{\overline{\varepsilon_1}^3}$$

$\delta$ : displacement from the position where the ball indenter 40 contact with the skin U1
$\hat{F}$ : pushing load of the specimen having finite thickness
$\phi$ : diameter of the ball indenter 40
$\nu$ : Poisson ratio of the skin U1
$B$ : factor which indicate influence of load which increase with the pushing
$\overline{\varepsilon}_1$ : corresponding pushing strain

It should be noted that, in a case in which displacement of a left end side 20b of the load cell 20 due to pressing of the ball indenter 40 cannot be ignored, the control unit 66 can be configured to correct the displacement, which is based on the driving pulse, by adding the displacement of the left end side 20b. In this case, the displacement of the left end side 20b and the output from the load cell 20 may be measured and stored in the storage unit 65 in advance.

The control unit 66 can then read the displacement of the left end side 20b from the storage unit 65 based on the output from the load cell 20.

In S7, the control unit 66 outputs a viscoelasticity measurement result on the display unit 62. Alternatively, in order to allow the measurer U to check the measurement result afterward, the control unit 66 can be configured to store the measurement result in the storage unit 65, so as to be readable according to an operation of the operation unit 61.

In S8, the control unit 66 terminates a set of processes.

All or some functions of: the operation unit 61, the display unit 62, the storage unit 65, and the control unit 66 can be realized by an external device such as a PC (personal computer) connected to the measuring apparatus 1.

Alternatively, both the measuring apparatus 1 and the external device can have these functions.

For example, for an operation of starting measurement performed on the operation unit 61, a switch for starting measurement can be provided in the measuring apparatus 1, and a button for submitting a command for starting measurement to communication software or the like can be provided in the external device. This allows the measurer to select the operation of starting measurement based on measurement scenes.

A circuit for communication between the measuring apparatus 1 and the external device is provided on the substrate 50. Connection between the measuring apparatus 1 and the external device can be realized either in a wired manner (RS-232C, USB, and the like) or in a wireless manner (Bluetooth (registered trademark), Wi-Fi (registered trademark), and the like).

As described above, the measuring apparatus 1 according to the present embodiment can improve the measuring precision by arranging the driving unit 30 on the movable end side of the load cell 20. Since the control unit 66 obtains the displacement of the ball indenter 40 based on the driving pulse of the pulse motor 31, a configuration of the measuring apparatus can be simplified.

(Second Embodiment)

A second embodiment of the present invention is described hereinafter.

In the description and drawings of the following embodiments, components with similar functions to those of the first embodiment are referred to by the same numerals or numerals with the same last two digits, and a detailed description thereof will be omitted accordingly.

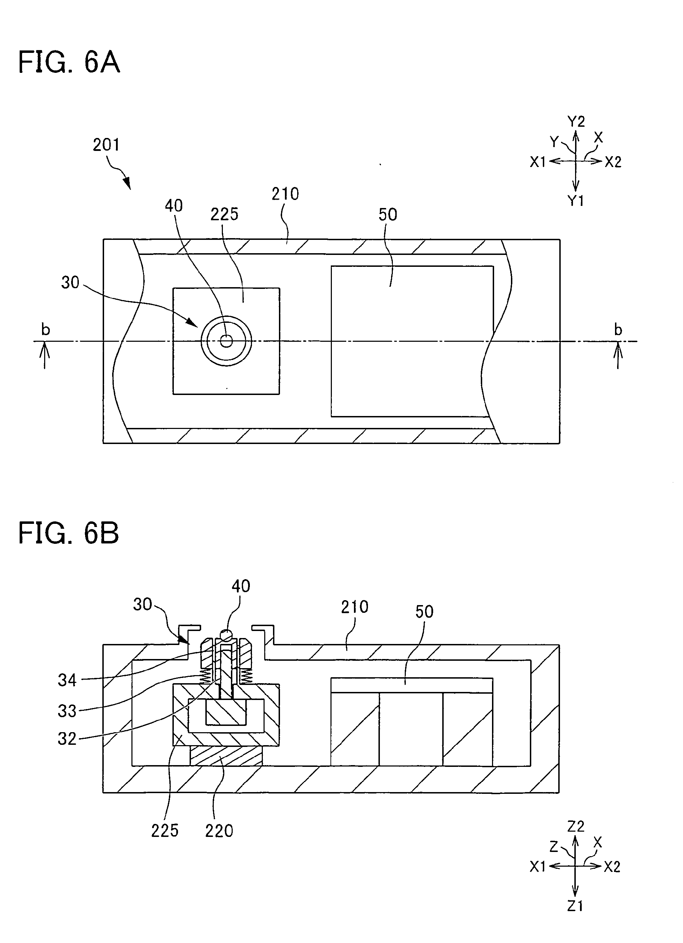

FIGS. 6A and 6B are a top view and a cross-sectional view of a measuring apparatus 201 of the second embodiment (corresponding to FIGS. 2A and 2B).

The measuring apparatus 201 is provided with a load sensor 220 and a chassis 225.

As the load sensor 220, a small-sized piezoelectric element in a cylindrical shape or the like is used. A lower face (fixed end side) of the load sensor 220 is fixed to a bottom face of a casing 210. To an upper face (movable end side) of the load sensor 220, the chassis 225 is fixed.

The chassis 225 functions as a base for attachment of a driving unit 30.

In the above-described configuration, in the measuring apparatus 201, the load sensor 220 measures the pushing load of the ball indenter 40 via the driving unit 30, as in the first embodiment.

As described above, since the measuring apparatus 201 uses the piezoelectric element as the load sensor 220, the entire apparatus can be reduced in size.

(Third Embodiment)

A third embodiment of the present invention is described hereinafter.

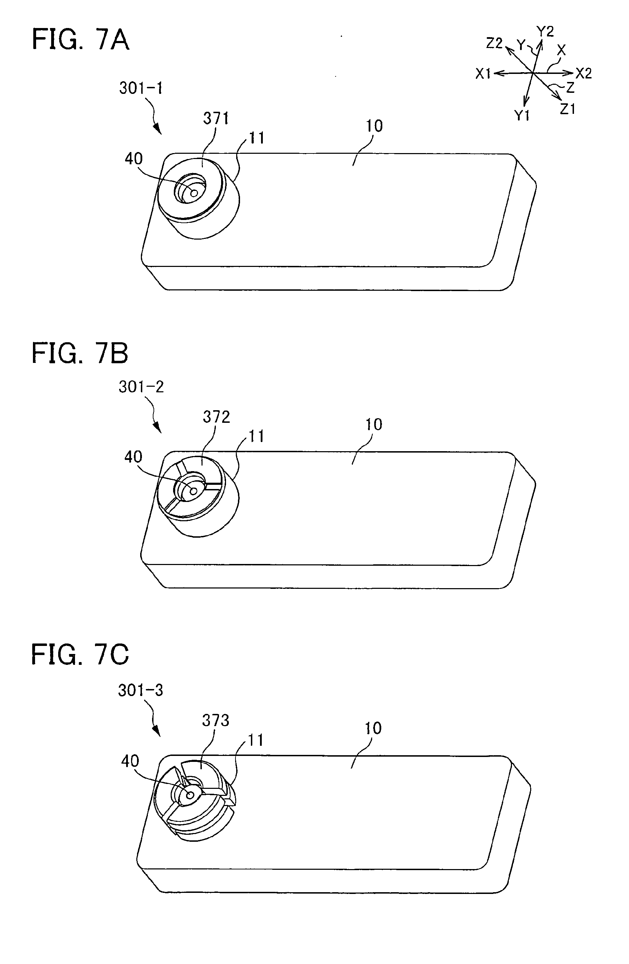

FIGS. 7A, 7B and 7C are perspective views of measuring apparatuses 301-1 to 301-3 of the third embodiment.

As shown in FIG. 7A, the measuring apparatus 301-1 is provided with an adhesive sheet 371.

The adhesive sheet 371 is provided at a top end of an annular part 11. The adhesive sheet 371 adheres to the skin during measurement. The measuring apparatus 301-1 can thus correct a contact angle of a ball indenter 40 with the skin, and can make the top end of the annular part 11 (surface contact part) adhere to the skin surface even when the measurer's hand or body shakes, thereby reducing influence of the shaking on the measurement.

As shown in FIG. 7B, the measuring apparatus 301-2 is provided with three pressure sensors 372.

The pressure sensors 372 are arranged in the top end of the annular part 11 at predetermined intervals. The pressure sensors 372 output pressure information being detected to a control unit 66 (see FIG. 4). The control unit 66 starts measurement when output differences of the three pressure sensors 372 are within a predetermined range.

As the result, the measuring apparatus 301-2 can start the measurement only when the contact angle of a ball indenter 40 is corrected to a value within a predetermined range. As the measurement can be performed with appropriate contact pressure between a surface contact part and the skin, measurement error due to hardened skin as a result of excessive contact pressure can be curtailed.

The control unit 66 can also be configured to alert (notify) the measurer of the possibility of an incorrect measurement result with sound or light, in a case in which the output differences of the pressure sensors 372 or the gravity centers of the pressure sensors 372 are not within a predetermined range.

Alternatively, the pressure sensors 372 can be a single annular member, not the three separate members. Also in this case, since the control unit 66 is configured to start the measurement when the sensor output is within an appropriate range, the measuring apparatus 301-2 can start the measurement only when the contact angle of the ball indenter 40 is corrected to a value within a predetermined range. As the measurement can be performed with appropriate contact pressure between the surface contact part and the skin, measurement error due to hardened skin as a result of excessive contact pressure can be curtailed. Furthermore, this configuration allows reduction in the number of components.

As shown in FIG. 7C, the measuring apparatus 301-3 is provided with three switches 373.

The switches 373 are arranged in the top end of the annular part 11 at predetermined intervals. The switches 373 are arranged to activate an electrical contact thereinside upon depression by a predetermined amount to the retraction direction Z1, and to output a signal to the control unit 66 (see FIG. 4). The control unit 66 starts the measurement only when the signal is output from the three switches 373.

As the result, the measuring apparatus 301-3 can start the measurement only when the contact angle of the ball indenter 40 is corrected to a value within a predetermined range.

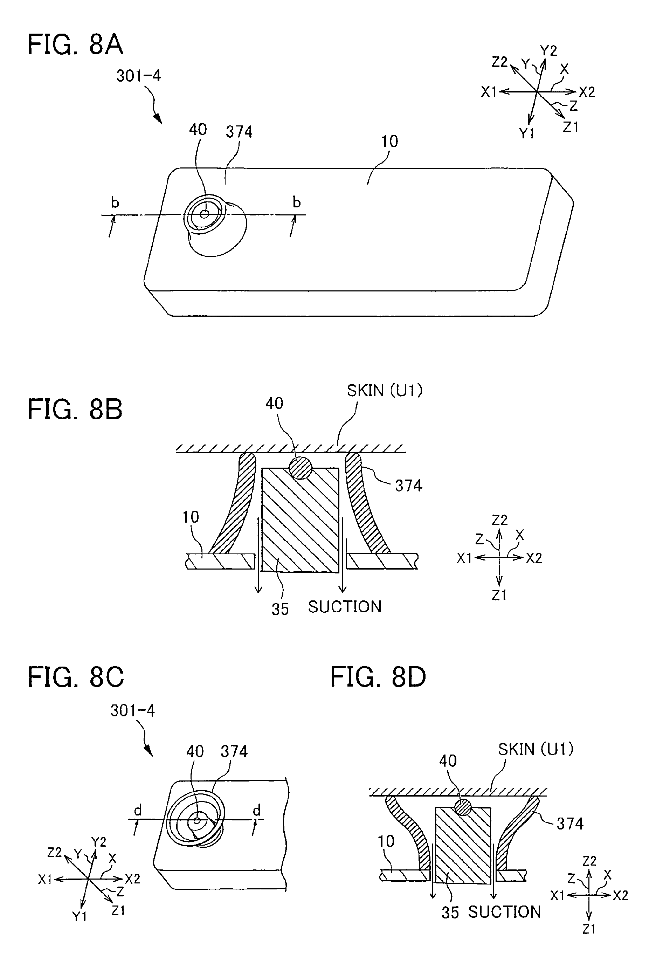

FIGS. 8A-8D are perspective views and cross-sectional views of the measuring apparatus 301-4 of the third embodiment.

FIG. 8A is a perspective view of the measuring apparatus 301-4.

FIG. 8B is an enlarged cross-sectional view taken along the line b-b of FIG. 8A.

FIGS. 8C and 8D are a perspective view and a cross-sectional view of other aspects of the measuring apparatus 301-4 of the third embodiment.

As shown in FIGS. 8A and 8B, the measuring apparatus 301-4 is provided with a suction nozzle 374 instead of the annular part.

The measuring apparatus 301-4 activates a suction pump or the like (not illustrated) to suction the air, in a state in which the suction nozzle 374 is in contact with the skin U1, to thereby bring the skin U1 into close contact with an edge of the suction nozzle 374.

Accordingly, the measuring apparatus 301-4 can correct the contact angle of the ball indenter 40 to the skin U1.

The measuring apparatus 301-3 can make the top end of the suction nozzle 374 (surface contact part) adhere to the skin surface even when the measurer's hand or body shakes, thereby reducing influence of the shaking on the measurement.

It should be noted that the suction nozzle 374 can be configured to increase in diameter as it comes closer to the top end thereof, as shown in FIGS. 8C and 8D.

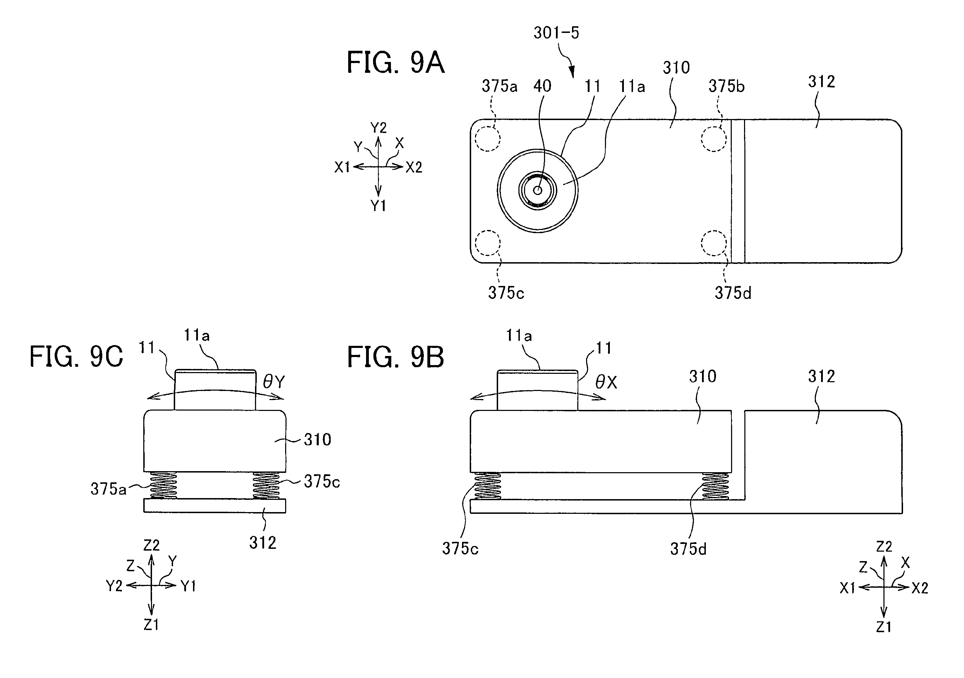

FIGS. 9A-9C are three-view drawings of the measuring apparatus 301-5 of the third embodiment.

The measuring apparatus 301-5 includes the casing 310, a grip part 312, and four springs 375a to 375d.

The casing 310 is a member similar to the casing 10 of the first embodiment and houses a load cell, a driving unit and the like as in the first embodiment.

The grip part 312 is a housing that supports the casing 310. During measurement, the measurer places the grip part 312 in his/her palm or grasps the grip part 312 to put the surface contact part 11a against the skin.

The springs 375a to 375d are helical springs provided between the casing 310 and the grip part 312. Lower ends of the springs 375a to 375d are fixed to the grip part 312. Upper ends of the springs 375a to 375d are fixed to bottom corner portions of the casing 310.

As the springs 375a to 375d are compressed and extended, the casing 310 is rotatably supported in rotational directions $\theta$X and $\theta$Y with respect to the grip part 312.

As a result, in the measuring apparatus 301-5, when the surface contact part 11a is in contact with the skin, the casing 310 rotates to correct the contact angle of the ball indenter 40 with respect to the skin surface. In addition, this can absorb the vibration between the surface contact part 11a and the skin due to hand shaking and body movement during measurement, thereby reducing the influence of vibration on the measurement.

Furthermore, the measurer does not hold the casing 310 directly during measurement. As a result, the measuring apparatus 301-5 can reduce strain of the casing 310 due to holding by the measurer, thereby reducing strain of the load cell caused by the strain of the casing 310. As a result, the measuring apparatus 301-5 can further improve the measuring precision.

The springs 375a to 375d can be other elastic members. For example, a blade spring and rubber can be used instead of the springs 375a to 375d.

Furthermore, by providing a sensor that detects compression of the springs exceeding a preset amount, the control unit 66 (see FIG. 4) can be configured to not perform measurement in response to output from the sensor, and if an alert (notice) is given to the measurer concerning the possibility of an incorrect measurement result with sound or light, it is possible to curtail measurement error due to hardened skin as a result of excessive contact pressure.

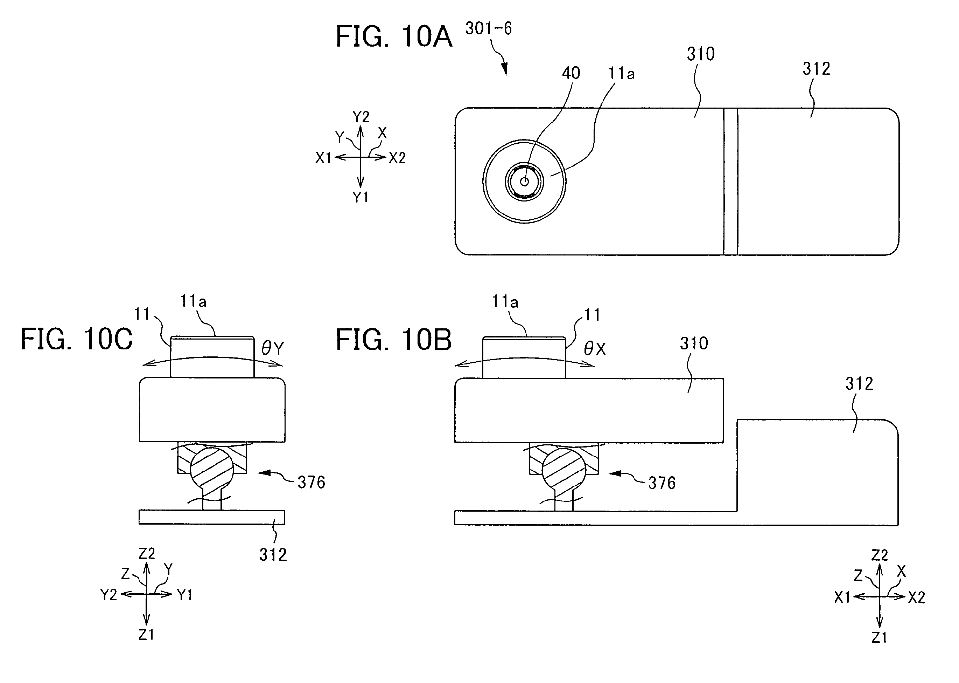

FIGS. 10A-10C are three-view drawings of the measuring apparatus 301-6 of the third embodiment.

The measuring apparatus 301-6 includes the casing 310, the grip part 312, and a ball joint 376.

While the measuring apparatus 301-5 is configured such that the springs 375a to 375d connect the casing 310 with the grip part 312, the measuring apparatus 301-6 is configured such that the connection is realized by the ball joint 376.

As a result, the measuring apparatus 301-6 can correct the contact angle of the ball indenter 40 with respect to the skin surface, reduce the influence of vibration between the surface contact part 11a and the skin, and reduce strain of the casing thereby improving the measurement precision, by not making the measurer directly hold the casing that houses the load cell.

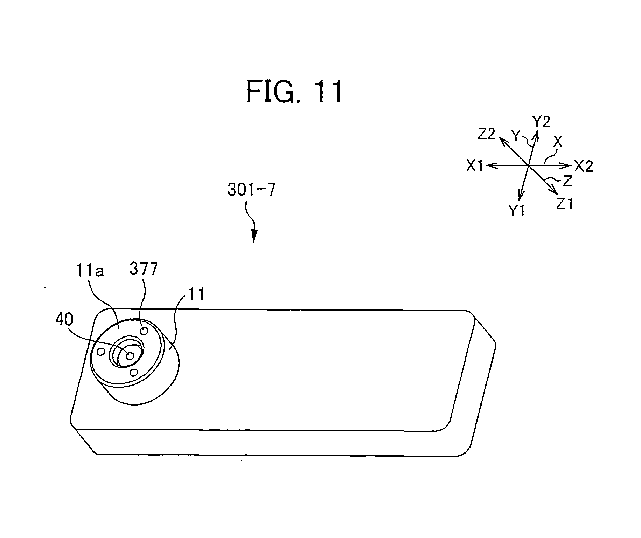

FIG. 11 is a perspective view of the measuring apparatus 301-7 of the third embodiment.

The measuring apparatus 301-7 is provided with three projections 377.

The projections 377 are provided on the surface contact part 11a at a top end of the circular part 11, at equal intervals. The projections 377 project from the surface contact part 11a.

The measurer can thus check a sensation of the projections 377 in contact with the skin to thereby correct the degree of contact with the surface contact part 11a.

As a result, the measuring apparatus 301-7 can correct the contact angle of the ball indenter with respect to the skin surface.

In addition, by providing an acceleration sensor and a load sensor (load difference detection unit), the influence of load difference due to hand shake and the like can be suppressed, thereby improving measurement precision. The load sensor detects load variation with respect to an initial load at the beginning of measurement. The initial load is defined by an angle of the measuring apparatus (x, y, z directions). Hand shaking changes the angle from the initial position and the initial load, leading to an error in a load value. The acceleration sensor and the load sensor detect and correct such an error.

For example, in a case in which the acceleration sensor is provided, change of the initial load is estimated and corrected based on the initial position and acceleration information relating to hand shaking.

On the other hand, in a case in which the load sensor is provided, in addition to the load sensor for measurement, a load sensor for correction should be provided of which detection direction is the same as that of the load sensor for measurement. The initial load of the load sensor for correction is adjusted by making it the same as that of the load sensor for measurement, or by multiplying the load value by a coefficient. By subtracting an output of the load sensor for correction from an output of the load sensor for measurement, a measurement error caused by angle variation from the initial position due to hand shaking and the like can be cancelled, and only a load applied on the ball indenter 40 can be extracted. According to the above-described embodiments, it is only required to drive the ball indenter 40, which is light in weight, without driving the load detection unit, which is heavy in weight. This allows reduction in size and weight of the driving unit. As a result, the influence of shaking of the hand of the measurer holding the measuring apparatus during measurement can thus be reduced. In addition, since the driving unit can function with a reduced driving force, driving electricity and power consumption can be reduced.

Furthermore, the distance between the driving unit and the measurement target can be reduced. This can suppress axial runout of the driving unit due to shaking of the hand and the like, eliminating the need for components such as a bearing and coupling for controlling the axial runout, and cost reduction can be realized. Moreover, there is no influence of slide friction generated by the bearing and the like, and measuring precision can be improved.

In addition, since the load sensor can be firmly fixed to the housing of the measuring apparatus, the measurement precision of the load sensor can be improved.

According to the above-described embodiments, the control unit can calculate viscoelasticity of the measurement target based on an output from the load detection unit and displacement of the indenter at the pressure position of the indenter.

According to the above-described embodiments, since the control unit obtains the displacement of the indenter based on the driving pulse of the pulse motor, configuration of the measuring apparatus can be simplified.

According to the above-described embodiments, since the displacement of the load detection unit itself is added as the displacement of the indenter, the measurement precision can further be improved.

According to the above-described embodiments, since the contact angle of the indenter is corrected, the measurement precision can further be improved.

According to the above-described embodiments, since the pressure of contact between the surface contact part and the measurement target is appropriately corrected, precision of load detection and displacement obtention can be improved, thereby improving measurement precision.

According to the above-described embodiments, since vibration between the surface contact part and the measurement target, stable load detection and displacement obtention can be realized even with hand shaking and body movement of the measurer, thereby improving measurement precision.

The embodiments of the present invention have been described above; however, the present invention is not limited thereto and various changes can be made such as modifications presented below, and such changes are also within the scope of the present invention. In addition, the effects mentioned in the embodiments are merely examples of most desirable effects provided by the present invention, and the present invention is not limited thereto. It should be noted that the above-described embodiments and the modification can be used in combination accordingly, of which a detailed description is omitted.

(Fourth Embodiment)

Next, a fourth embodiment of the present invention is described hereinafter.

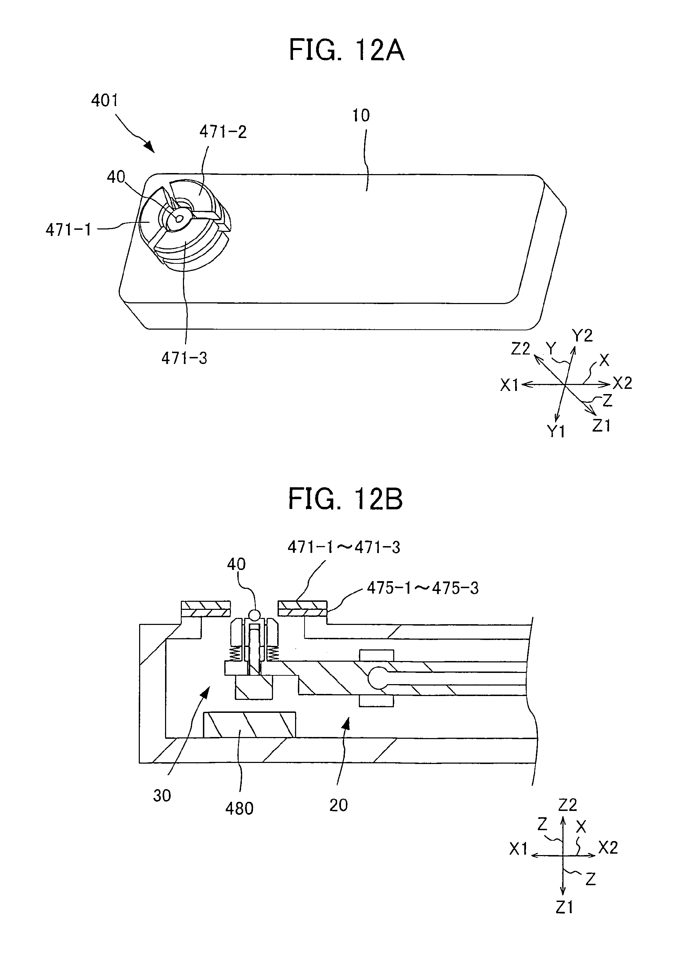

FIGS. 12 are a perspective view and a cross-sectional view of a measuring apparatus 401 of the fourth embodiment.

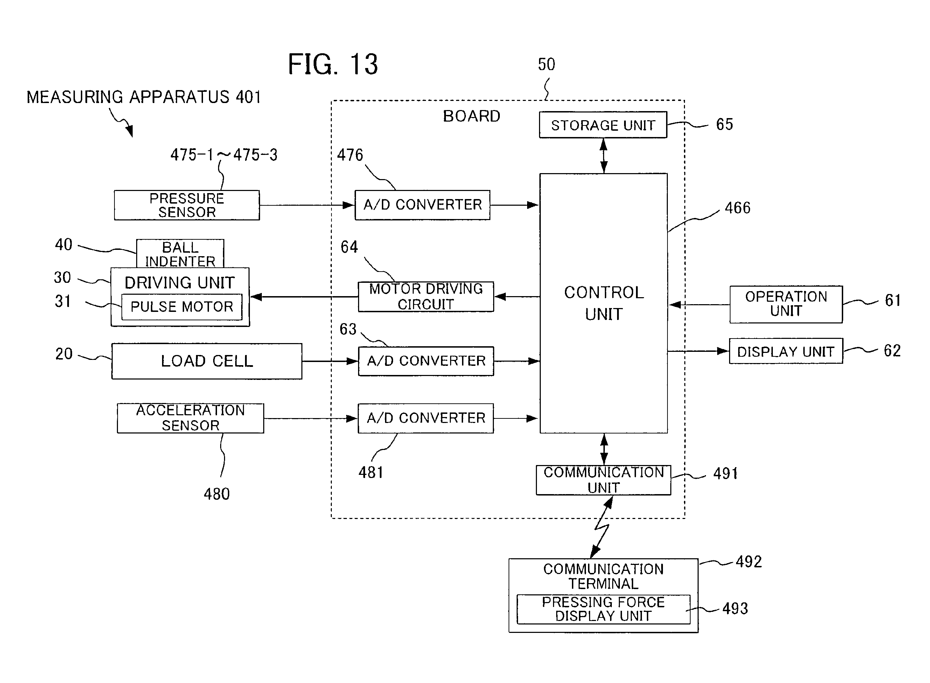

FIG. 13 is a block diagram of the measuring apparatus 401 of the fourth embodiment.

The measuring apparatus 401 is similar to the measuring apparatus 301-2 described with reference to FIG. 7 and includes three pressure sensors 475 (475-1 to 475-3).

As shown in FIGS. 12 and FIG. 13, the measuring apparatus 401 includes the pressure sensors 475 (pressing force detecting unit), an acceleration sensor 480 (inclination detecting unit), a communication unit 491, a communication terminal 492, and a control unit 466.

The pressure sensors 475 (475-1 to 475-3) detect the pressing force onto a skin surface from a surface (surface contact part) of the annular part 471 (471-1 to 471-3). As the pressure sensors 475, for example, a sensor with a similar structure to the load cell 20 or a sensor with a piezoelectric element is applicable. As the inclination detecting unit, not just the acceleration sensor but also an inclination sensor, a load cell, or the like is applicable.

The pressure sensors 475-1 to 475-3 are disposed right below the annular parts 471-1 to 471-3. The pressure sensors 475-1 to 475-3 detect the pressing force on skin applied by the annular parts 471-1 to 471-3.

The detecting direction of the pressure sensors 475 is a thickness direction Z. The output of the pressure sensors 475 is subjected to A/D conversion through an A/D converter 476, and then output to the control unit 466.

The shape of the annular part 471 is not limited to the annular shape as long as the shape is like a ring (frame) surrounding the ball indenter 40. For example, this shape may be rectangular or triangular.

The acceleration sensor 480 detects the inclination of the detecting direction of the load cell 20 and the inclination of the detecting direction of the pressure sensor 475. The acceleration sensor 480 is an acceleration sensor 480 utilizing a piezoelectric element or the like. The direction in which the acceleration sensor 480 detects the acceleration is the thickness direction Z, which is the same as the detecting direction of the load cell 20 and the pressure sensor 475. Therefore, the acceleration sensor 480 can detect the inclination of these detecting directions from the vertical direction. The output of the acceleration sensor 480 is subjected to the A/D conversion through an A/D converter 481, and then output to the control unit 466.

The acceleration sensor 480 is disposed in the vicinity of the load cell 20. Therefore, the acceleration sensor 480 can detect the acceleration in the vicinity of the load cell 20, so that the inclination of the detecting direction of the load cell 20 can be accurately detected.

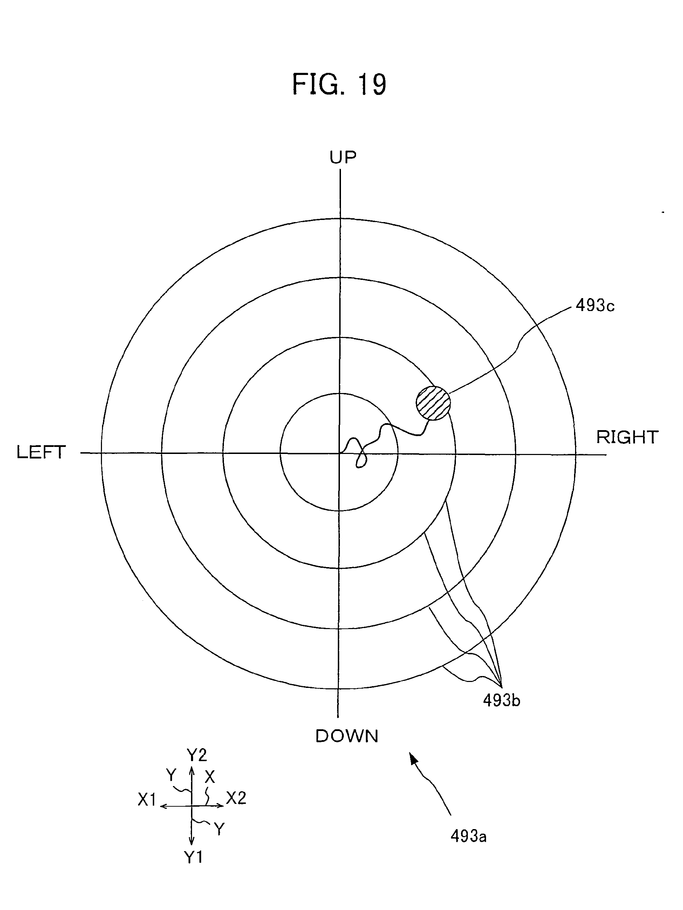

The communication unit 491 transmits a pressing force information display screen 493a (see FIG. 19) to the communication terminal 492. This pressing force information display screen 493a shows information on the pressing force of the annular part 471. The communication unit 491 includes an RF circuit, an antenna, etc. for wireless communication.

The communication terminal 492 corresponds to a portable information terminal, a personal computer, a tablet terminal, or the like. The communication terminal 492 has a function of receiving the pressing force information display screen 493a through wireless communication with the communication unit 491.

The communication terminal 492 includes a pressing force display unit 493.

The pressing force display unit 493 is a display device such as a liquid crystal display device. The pressing force display unit 493 displays the pressing force information display screen 493a (see FIG. 19). A measurer can perform the measurement while observing the display of the pressing force display unit 493. In this case, since the measuring apparatus 401 and the communication terminal 492 are separate bodies, a subject can observe the pressing force display unit 493 even when the subject measures his/her own cheek alone, for example.

It should be noted that the communication unit 491 and the communication terminal 492 may transmit and receive the image information through wired communication using a communication cable, for example. The display unit 62 may also serve as the pressing force display unit 493. In addition to the similar process to the above embodiment, the control unit 466 performs various processes as later described.

Zero point of load cell 20

The zero point of the load cell 20 of the measuring apparatus 401 is described.

(Measurement error in the inclination of the measuring apparatus)

First, the measurement error in the inclination of the measuring apparatus is described.

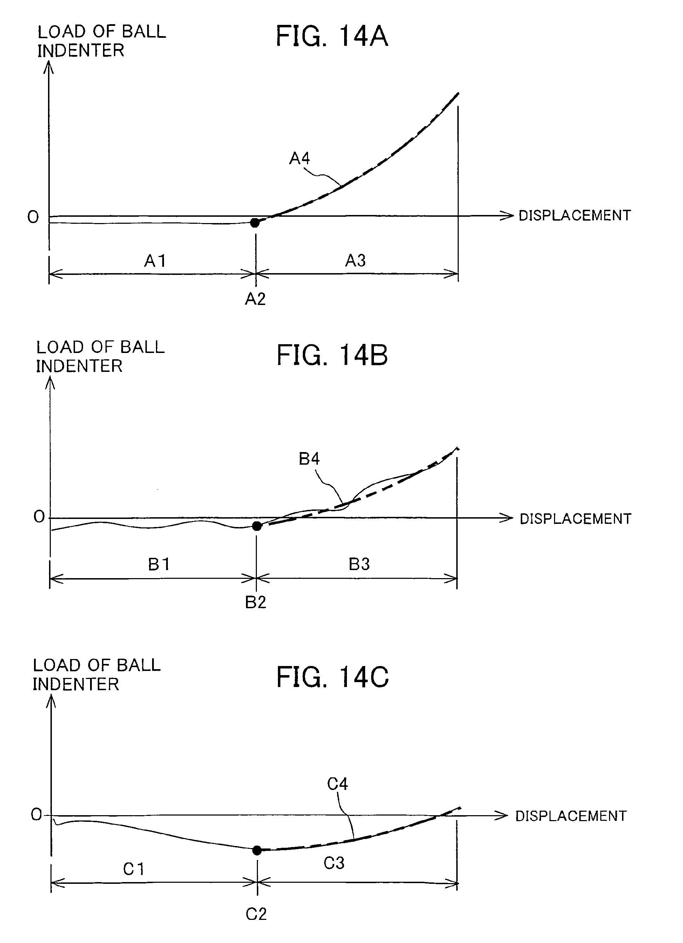

FIGS. 14 are graphs for describing the measurement error in the inclination of the measuring apparatus.

FIG. 14A is a graph representing the measurement result in the case in which the inclination of the measuring apparatus is constant and the measurement is performed correctly.

FIG. 14B is a graph representing the measurement result in the case in which the measuring apparatus is shaken due to the shake of a subject.

FIG. 14C is a graph representing the measurement result in the case in which the measuring apparatus is gradually inclined due to the gradual inclination of a subject.

As indicated in FIG. 14A, in the correct measurement, the measured value based on the load cell 20 is almost zero and is not varied in a period A1 after the start of the measurement and before the contact between the ball indenter 40 and the skin. This is because the zero point (detection value reference point) is stable. It should be noted that the zero point is a reference point at which the load of the ball indenter 40 should be determined as 0 gf. In other words, the zero point is the output value of the load cell 20 corresponding to a load of the ball indenter 40 of 0 gf.

In this case, the load variation at the contact (contact point A2) between the ball indenter 40 and the skin can be determined with high accuracy. The zero point is stable in a period A3 after the contact (after the contact point A2 to the measurement end). Thus, the graph of the measured values is a curve.

Based on the measured values in the period A3 after the contact, the control unit 466 calculates a Young's modulus (viscoelasticity) by performing parameter fitting on "Equations 31 and 34 in PCT International Application Publication No. WO 2010/084840" described in the first embodiment. The function based on the above Equations is expressed on the graph as a fitting curve A4 (curve corresponding to the function based on the Equations).

The fitting curve A4 is drawn with a two-dot chain line in the graph. The fitting curve A4 substantially coincides with the curve representing the measured values. That is, the Young's modulus calculated through correct measurement is accurate.

For accurately creating the fitting curve, it is important to determine the position of the contact point accurately. Therefore, in order to improve the measurement accuracy, it is important to determine the contact point more accurately.

As depicted in FIG. 14B, in the occurrence of the shake, the measured values vary depending on the shake period or the like of the subject in a period B1 before the contact.

Such a shake is caused by the breathing of the subject, for example. Such variation in measured value is caused when, for example, the variation in inclination of the measuring apparatus 401 leads to variation of the load on the load cell 20 because of the initial load of the driving unit 30, the ball indenter 40, or the like, thereby varying the zero point.

In this case, the determination accuracy of the contact point B2 between the ball indenter 40 and the skin deteriorates. Moreover, the measured values vary in a period B3 after the contact. A fitting curve B4 does not coincide with the curve line representing the measured values.

For this reason, even though the Young's modulus is calculated, this modulus is not accurate.

As depicted in FIG. 14C, even when the subject is inclined gradually, the zero point gradually varies in response to the inclination in a period C1 before the contact. This inclination is caused when, for example, the body of the subject is gradually inclined due to the pressing of the measuring apparatus 401 on the cheek.

Therefore, like in FIG. 14B, the accuracy of the control unit 466 for determing the contact point C2 between the ball indenter 40 and the skin deteriorates. Even after the contact C3, the measurement error is caused. The fitting curve C4 coincides with the curve line representing the measured values; however the zero point varies. For this reason, even though the Young's modulus is calculated, this modulus is not accurate.

(Process for correcting zero point of load cell 20)

A process for correcting the zero point of the load cell 20 is described.

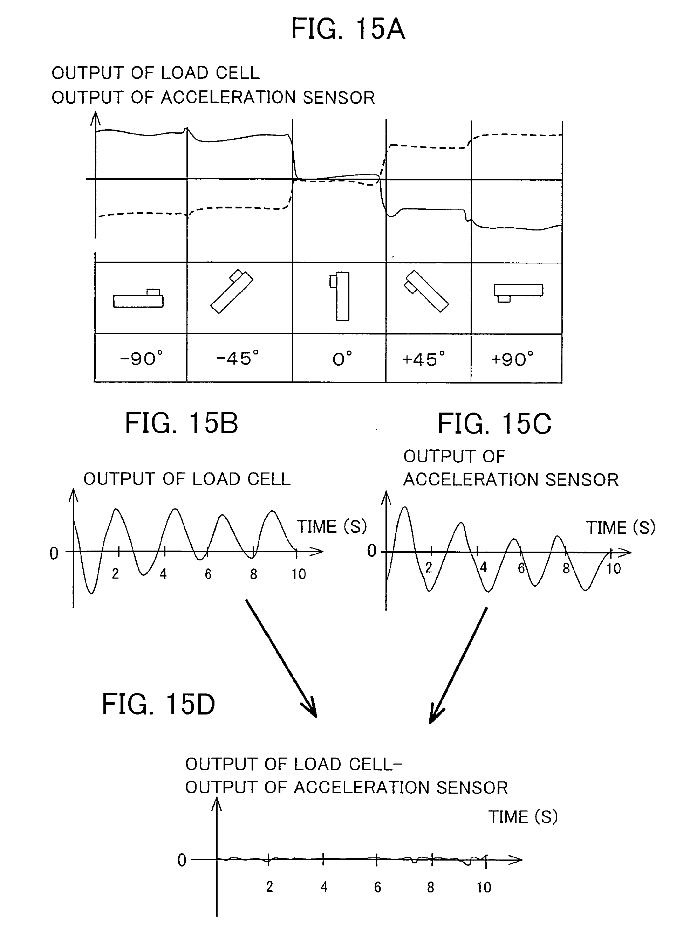

FIGS. 15 are graphs representing the output of the load cell 20 and the acceleration sensor 48 of the fourth embodiment.

FIG. 15A is a graph for describing the comparison between the output of the load cell 20 and the output of the acceleration sensor 480 when the measuring apparatus 401 is gradually inclined.

As depicted in FIG. 15A, the output of the load cell 20 and the output of the acceleration sensor 480 were compared while the detecting direction of the load cell 20 is varied in five stages as follows.

· Inclination of -90° : detecting direction is upward
· Inclination of -45° : detecting direction is obliquely upward at the angle of 45°
· Inclination of 0° : detecting direction is horizontal
· Inclination of +45° : detecting direction is obliquely downward at the angle of 45°
· Inclination of +90° : detecting direction is downward

A solid line represents the output of the load cell 20.

A dotted line represents the output of the acceleration sensor 480. The output of the acceleration sensor 480 was adjusted to be the same as the output of the load cell 20 when the inclination is 0° . The correction was performed by integrating the coefficient so that the variation range of the output of the acceleration sensor 480 between the inclination of -90° and the inclination of +45° was equal to the variation range of the load cell 20. In other words, the output of the acceleration sensor 480 was amplified.

By the way, the load cell 20 detects the loads of the ball indenter 40, the driving unit 30, etc. corresponding to the inclination of the measuring apparatus 401. The acceleration sensor 480 detects the acceleration that corresponds to the inclination of the measuring apparatus 401.

The detecting direction of these sensors is the same (thickness direction Z). Therefore, the output of these sensors varies similarly in accordance with the inclination of the measuring apparatus 401.

In view of this, the control unit 466 performs the process for correcting the zero point of the load cell 20 by offsetting (subtracting or adding) the output of the load cell 20 with the adjusted force of the acceleration sensor 480.

In this embodiment, the output value of the load cell 20 and the output value of the acceleration sensor 480 are corrected so that the positive and negative properties thereof become opposite; therefore, the both may be summed up. The process for correcting the zero point is easy because the process merely requires such simple calculation.

FIG. 15B and FIG. 15C represent the output of the load cell 20 and the acceleration sensor 480 in the shake of the subject.

FIG. 15D is a graph representing the result of correcting the zero point on the basis of the output shown in FIG. 15B and FIG. 15C.

It should be noted that the graphs of FIG. 15B to FIG. 15D are obtained by performing the measurement before the ball indenter 40 is brought into contact with the skin.

As shown in FIG. 15B, the output of the load cell 20 varies depending on the shake period of the subject. As shown in FIG. 15C, the output of the acceleration sensor 480 similarly varies depending on the shake period of the subject.

Due to the above correction, the output of the acceleration sensor 480 has the similar amplitude to the output of the load cell 20.

The control unit 466 corrects the zero point of the load cell 20 by adding the output value (FIG. 15C) of the acceleration sensor 480 to the output value (FIG. 15B) of the load cell 20.

As shown in FIG. 15D, the output after the correction is substantially zero; thus, it has been confirmed that the zero point can be corrected.

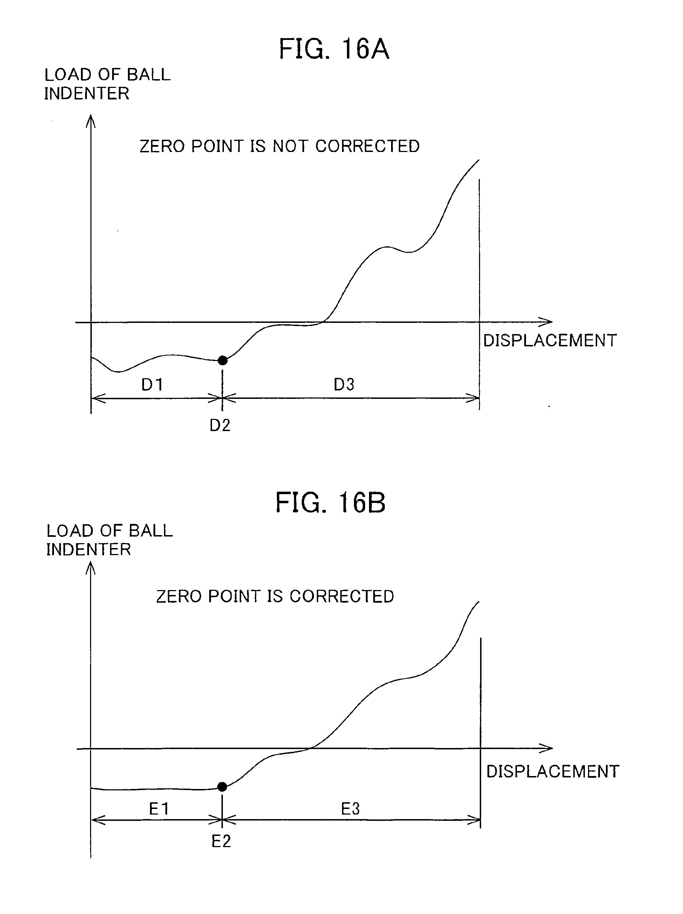

FIGS. 16A and 16B are the graphs representing the results of measuring the load and displacement of the skin in the shake of the subject.

FIG. 16A represents the measurement result when the zero point of the load cell 20 is not corrected.

FIG. 16B represents the measurement result when the zero point of the load cell 20 is corrected.

As shown in FIG. 16A, in the shake of the subject, the measurement results are influenced by the shake of the subject both in a period D1 before the contact between the ball indenter 40 and the skin and in a period D3 after the contact, in a manner similar to FIG. 14B. Further, the determination of a contact point D2 is not accurate.

After the correction of the zero point of the load cell 20 as shown in FIG. 16B, the measured values of the load are stable in a period E1 before the contact. Further, in a period E3 after the contact, the influence of the shake of the subject is reduced. The determination of a contact point E2 is also accurate.

Thus, the measuring apparatus 401 performs the zero point correcting process for the load cell 20; therefore, even in the shake of the subject, the contact between the ball indenter 40 and the skin can be accurately determined, and the measurement can be accurately performed even after the contact.

The similar effect can be achieved even when the subject is gradually inclined (see FIG. 14C), though the description is omitted.

Correcting process for the pressure sensor 475

The correcting process for the pressure sensor 475 is described.

(Process for correcting zero point of the pressure sensor 475)

The correction of the zero point for the pressing force detection of the measuring apparatus 401 is described.

The measuring apparatus 401 utilizes the output of the acceleration sensor 480 for the zero point correction (detection reference point) for the pressure sensor 475 in addition to the zero point correction for the load cell 20.

The detecting directions of the load cell 20, the pressure sensor 475, and the acceleration sensor 480 are the same (thickness direction Z). Therefore, the acceleration sensor 480 can detect the inclination of the detecting direction of the pressure sensor 475.

The zero point correction for the pressure sensor 475 can be performed based on the following method, for example.

Even if the measuring apparatus 401 is inclined, the load of the annular part 471 and the like applied to the pressure sensor 475 can be calculated based on the inclination (angle) of the measuring apparatus 401.

Therefore, the load of the annular part 471 and the like applied to the pressure sensor 475 in a non-load state at -90° in FIG. 15A is measured in advance, and this load information is stored in the storage unit 65. The control unit 466 calculates the change in output of the pressure sensor 475 along with the inclination of the measuring apparatus 401 on the basis of the output of the acceleration sensor 480 and the load information in the storage unit 65. Then, the control unit 466 corrects the zero point by subtracting the change in output from the output of the pressure sensor 475.

It should be noted that the output of the acceleration sensor 480 corresponds to the amount of correction of the zero point. For this reason, the zero point correction may be performed in such a way that the inclination of the measuring apparatus 401 and the amount of correction of the zero point are stored in the storage unit 65 after being associated with each other. In this case, the control unit 466 may read out the amount of correction of the zero point by referring the storage unit 65 on the basis of the output of the acceleration sensor 480 and subtract the amount from the output of the pressure sensor 475.

Thus, even if the inclination is unstable because a measurer measures while holding the measuring apparatus 401 with a hand, the measuring apparatus 401 can measure the pressing force on the skin accurately.

(Process for making pressing force appropriate)

The process for making the pressing force of the measuring apparatus 401 appropriate is described.

Human skin has a property of, as the pressing force increases, having larger Young's modulus due to hardened skin.

Therefore, as the pressing force of the annular part 471 for pressing the human skin increases, the output of the load cell 20 tends to increase and the displacement of the ball indenter 40 tends to decrease. The process for making the  pressing force appropriate is a process for making this pressing force appropriate.

Based on the output of the three pressure sensors 475-1 to 475-3, the control unit 466 drives the driving unit 30 to measure the load and displacement of the ball indenter 40 only when the total of the pressing force of the annular parts 471-1 to 471-3 for pressing the skin is within a predetermined range. When the total pressing force is out of the predetermined range after the contact between the ball indenter 40 and the skin, the measurement is cancelled.

Thus, the measuring apparatus 401 can perform the measurement only when the total pressing force is within the predetermined range.

As the predetermined range, for example, a total pressing force of the three annular parts 471-1 to 471-3 of "0 to 200 gf" may be employed.

Thus, the measuring apparatus 401 can perform the measurement only when the pressing force is appropriate.

(Process for homogenizing pressing force distribution)

The process for homogenizing the pressing force distribution of the measuring apparatus 401 is described.

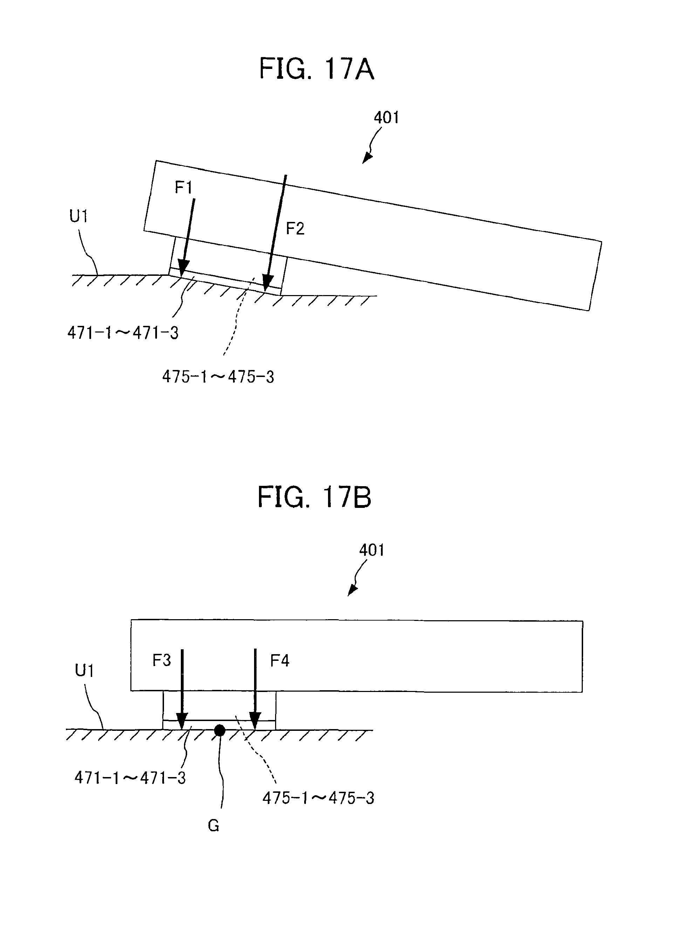

FIGS. 17 are explanatory views illustrating inhomogeneous and homogeneous states of the pressing force distribution of the annular part 471 of the fourth embodiment.

When the annular part 471 is brought into contact with skin U1 in the inclined manner as shown in FIG. 17A, the pressing force distribution of the annular part 471 becomes  inhomogeneous (see pressing force F1 and F2), in which case the surface layer of the skin U1 is pulled. In this state, the human skin U1 cannot be measured accurately.

Based on the output of the three pressure sensors 475-1 to 475-3, the control unit 466 drives the driving unit 30 to measure the load and the displacement of the ball indenter 40 only when the relative value of the pressing force of the annular parts 471-1 to 471-3 for pressing the skin (the value of another pressing force relative to the value of each pressing force) is within the predetermined range. The measurement is cancelled when the relative value of the pressing force is out of the predetermined range after the contact between the ball indenter 40 and the skin.

For example, in the case where the predetermined range is within 10 gf, when the pressing force of the annular parts 471-1 to 471-3 is 25 gf, 24 gf, and 26 gf, respectively, the relative value of the pressing force is within the predetermined range; therefore, the control unit 466 starts the measurement. Meanwhile, when the pressing force of the annular parts 471-1 to 471-3 is 50 gf, 15 gf, and 10 gf, respectively, the relative value of the pressing force is out of the predetermined range; therefore, the control unit 466 cancels the measurement.

Thus, the measuring apparatus 401 can perform the measurement only when the relative value of the pressing force is within the predetermined range.

When the relative value of the pressing force is within  the predetermined range as shown in FIG. 17B, the annular part 471 presses the skin U1 homogeneously (see pressing force F3 and F4). In this case, the measurement can be performed with the center point G of the operation of the pressing force close to the center of the annular part 471 (i.e., the position of the ball indenter 40).

Thus, the measuring apparatus 401 can perform the measurement with the surface layer of the skin U1 pulled homogeneously.

A set of processes of the measuring apparatus 401 is described.

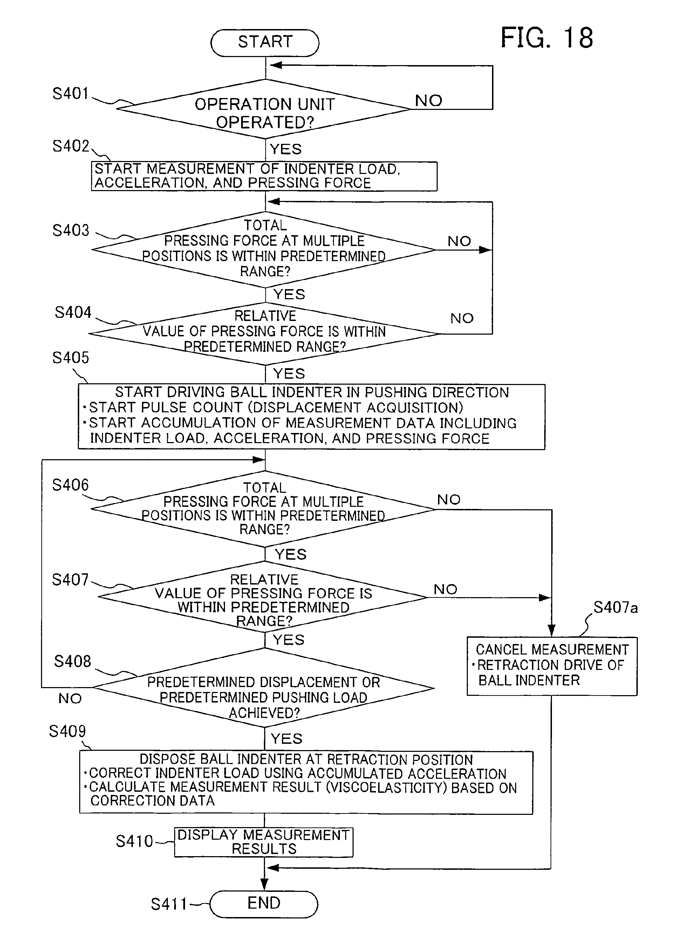

FIG. 18 is a flow chart illustrating a set of processes of the measuring apparatus 401 of the fourth embodiment.

FIG. 19 shows the pressing force information display screen 493a of the fourth embodiment.

S401 is similar to S1 of the first embodiment (see FIG. 5).

In S402, the control unit 466 starts to receive the output of the load cell 20, the pressure sensors 475-1 to 475-3, and the acceleration sensor 480 and starts to measure the load of the indenter, the acceleration (inclination), and the pressing force. The control unit 466 employs the pressing force which has been subjected to the zero point correcting process for the pressure sensor 475.

As shown in FIG. 19, the control unit 466 creates the pressing force information display screen 493a.

The pressing force information display screen 493a has a  circular scale 493b and an operation central point indicator 493c.

The circular scale 493b is formed by a plurality of concentric circles corresponding to the annular part 471. The center of the circular scale 493b corresponds to the center of the annular part 471 (i.e., the position of the ball indenter 40). The up, down, right, and left directions of the circular scale 493b correspond to a right and left direction X and a depth direction Y of the measuring apparatus 401.

The operation central point indicator 493c indicates the central point of the pressing force of the annular parts 471-1 to 471-3.

Therefore, the closer the operation central point indicator 493c is to the center of the circular scale 493b, the closer the center of the pressing force is to the position of the ball indenter 40. The state as above is appropriate for the measurement.

The control unit 466 displays the operation central point indicator 493c in a mode of being able to identify the total pressing force of the annular parts 471-1 to 471-3. This mode can be achieved by, for example, varying the color of the operation central point indicator 493c (blue if the total pressing force is small, green if it is appropriate, and red if it is large, for example), varying the color density, or varying the size of the display of the indicator, in accordance with the level of the total pressing force.

The control unit 466 may alternatively display the total  pressing force numerically. The control unit 466 sequentially creates the pressing force information display screen 493a until the measurement ends, and transmits the pressing force information display screen 493a to the communication terminal 492 by controlling the communication unit 491.

Upon the reception of the pressing force information display screen 493a, the communication terminal 492 displays the pressing force information display screen 493a on the pressing force display unit 493.

Thus, while observing the pressing force information display screen 493a in real time, a measurer can make the state of the pressing force appropriate by adjusting the inclination of the measuring apparatus 401. In other words, the measurer can adjust the inclination of the apparatus so that the operation central point indicator 493c comes inside a circle appropriate for the measurement (for example, the innermost circle of the circular scale 493b), or can adjust the degree of pressure application of the annular part 471 so that the operation central point indicator 493c has an appropriate color.

It should be noted that FIG. 19 shows the example of displaying the movement trace of the operation central point indicator 493c; however, the display of the trace may be omitted.

In S403, the control unit 466 performs the aforementioned process for making the pressing force appropriate. That is, the control unit 466 determines whether the total pressing  force of the annular parts 471-1 to 471-3 is within a predetermined range on the basis of the pressure sensors 475-1 to 475-3.

The processing advances to S404 if the control unit 466 determines that the total pressing force is within the predetermined range (S403: YES); meanwhile, if the control unit 466 determines that the total pressing force is out of the predetermined range (S403: No), this determination is repeated. In S404, the control unit 466 performs the process for homogenizing the pressing force distribution. That is, the control unit 466 determines whether the relative value of the pressing force of the annular parts 471-1 to 471-3 is within a predetermined range on the basis of the pressure sensors 475-1 to 475-3. The processing advances to S405 if the control unit 466 determines that the relative value is within the predetermined range (S404: YES); meanwhile, if the control unit 466 determines that the relative value is out of the predetermined range (S404: NO), the process subsequent to S402 is repeated.

The processing advances to S405 and the subsequent steps only when the pressing force is appropriate (S403: YES) and the pressing force distribution is homogeneous (S404: YES) after the processing of S403 and S404 of the measuring apparatus 401, thereby starting the measurement of the viscoelasticity of the skin.

In S405, the control unit 466 drives the driving unit 30 to start the driving of the ball indenter 40. The control unit  466 starts to accumulate (store) the measurement data including the pulse count of a pulse motor 31 (movement displacement of the ball indenter 40), the indenter load, the acceleration, and the pressing force in the storage unit 65.

In S406, the control unit 466 determines whether the total pressing force is within a predetermined range in a manner similar to S403. If the control unit 466 determines the total pressing force is within the predetermined range (S406: YES), the processing advances to S407; meanwhile, if the control unit 466 determines the total pressing force is out of the predetermined range (S406: NO), the processing advances to S407a.

In S407, the control unit 466 determines whether the relative value of the pressing force is within a predetermined range or not, in a manner similar to S404. If the control unit 466 determines the total pressing force is within the predetermined range (S407: YES), the processing advances to S408; meanwhile, if the control unit 466 determines the total pressing force is out of the predetermined range (S407: NO), the processing advances to S407a.

In S407a, the control unit 466 cancels the measurement and ends the processing (S411). In other words, the control unit 466 disposes the ball indenter 40 at a retraction position and ends the accumulation of the measurement data.

The processing advances to S407a when the state of the pressing force on the skin is not appropriate for the measurement (S406: NO, S407: NO). Therefore, in this case, the measuring apparatus 401 can cancel the measurement. In this case, the measurer may restart the measurement from the beginning.

In S408, the control unit 466 determines whether the predetermined displacement or the predetermined pushing load has been achieved or not, in a manner similar to S4 of the first embodiment (see FIG. 5). If the control unit 466 determines that the predetermined displacement or the like has been achieved (S408: YES), the processing advances to S409; meanwhile, if the control unit 466 determines that the predetermined displacement or the like has not been achieved (S408: NO), the processing returns to S406 and repeats the processing.

In S409, the control unit 466 corrects the load data of the ball indenter 40 on the basis of the measurement data accumulated in the storage unit 65. In other words, the control unit 466 corrects the load data of the ball indenter 40 using the acceleration data relative to the load data according to the zero point correcting process for the load cell 20. Then, on the basis of the corrected load data, the control unit 466 determines the contact point and the like, and further performs the parameter fitting, thereby calculating the Young's modulus. Thus, since the measuring apparatus 401 can measure the viscoelasticity using the highly reliable load data, the measurement accuracy can be improved.

In S410, the control unit 466 displays the measurement results (such as the graphs based on the corrected load data (see FIGS. 14, etc.) and the calculated Young's modulus) on the display unit 62.

In S411, the control unit 466 ends the set of processes.

As thus described, even though the measuring apparatus 401 of this embodiment is inclined because a measurer measures while holding the measuring apparatus 401 with a hand, the measuring apparatus 401 can achieve high measurement accuracy by correcting the zero point of each sensor. Moreover, since the measurement is performed when the contact state between the annular part 471 and the skin is appropriate, the measurement accuracy can be further improved.

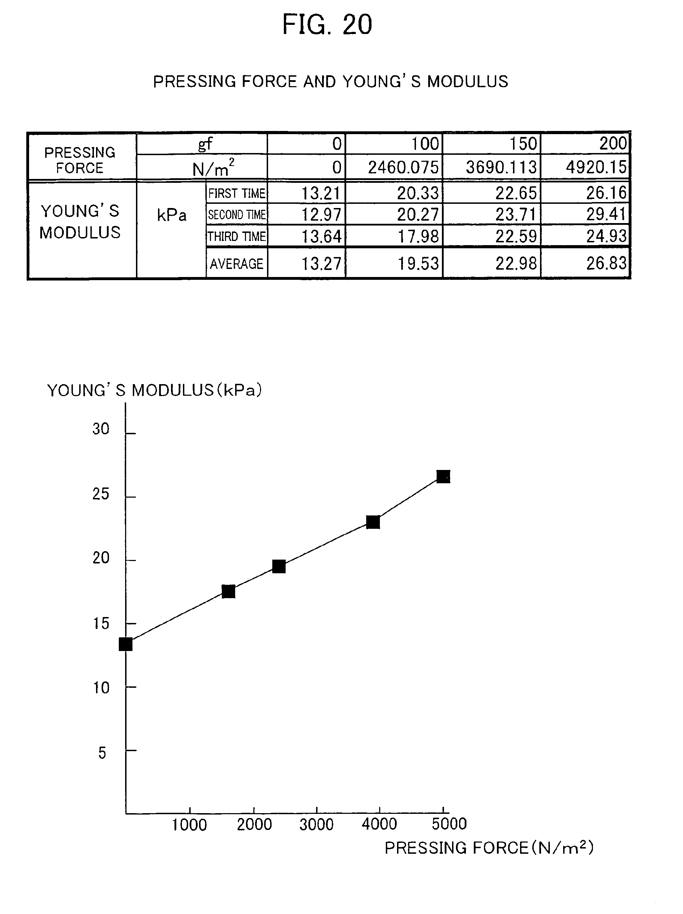

FIG. 20 represents the relation between the pressing force of the annular part 471 and the Young's modulus.

In the case of the handy type measuring apparatus 401 as shown in this embodiment, the measurement results are not stabilized unless the annular part 471 is pressed against the skin with a certain level of pressing force. In other words, in order to stabilize the load and the like of the ball indenter 40, it is necessary to press the annular part 471 against the skin with a certain level of pressing force.

Therefore, it is difficult to obtain the Young's modulus at a pressing force of 0 gf. Meanwhile, as depicted in FIG. 20, the pressing force and the Young's modulus are in proportion to each other.

In view of this, the apparatus measuring 401 can obtain the Young's modulus at a pressing force of 0 gf through the processing as below.

(1) The control unit 466 performs the measurement when the pressing force is, for example, 100 gf, 150 gf, and 200 gf on the basis of the output of the pressure sensors 475-1 to 475-3.
The number of measurements is not limited to three, and the pressing force is not limited to these numerals.
(2) The control unit 466 calculates the Young's modulus corresponding to the pressing force at these three points. The processing for this calculation may utilize the processing shown in FIG. 18.
(3) The control unit 466 obtains the approximation straight line or the approximation curved line as shown in FIG. 20 using the Young's modulus.
(4) The control unit 466 calculates the Young's modulus at a pressing force of 0 gf on the basis of the approximation straight line or the approximation curved line.

Thus, the measuring apparatus 401 can obtain the Young's modulus at a pressing force of 0 gf by obtaining the Young's modulus at other than a pressing force of 0 gf.

It should be noted that the Young's modulus to be obtained is not limited to the Young's modulus corresponding to a pressing force of 0 gf but may be the Young's modulus corresponding to a lower level of the pressing force than the pressing force measured at these three points.

According to the above-described embodiments, the detection reference point of the load detecting unit can be corrected even in the inclination of the measuring apparatus.

Thus, the contact point between the indenter and the measurement target can be accurately determined, and even after their contact, the viscoelasticity can be accurately measured.

According to the above-described embodiments, the detection reference point can be corrected by merely offsetting the output of the load detecting unit with the amount corresponding to the acceleration detected by the inclination detecting unit; thus, the correcting process is easy.

According to the above-described embodiments, the pressing force at the surface contact part is detected; therefore, the viscoelasticity can be measured when the state of the pressing force at the surface contact part is appropriate.

According to the above-described embodiments, the measurement is performed using the output of the load detecting unit or the like when the pressing force at the surface contact part is within the predetermined pressure; therefore, the change in viscoelasticity of the measurement target due to the change in pressing force can be reduced.

Further, even in multiple measurements, the measurement conditions can be homogenized.

According to the above-described embodiments, the viscoelasticity can be measured in a state that the annular parts press the measurement target homogeneously.

According to the above-described embodiments, the zero point of the pressing force detecting unit can be corrected even in the inclination of the measuring apparatus.

Thus, even though a measurer measures while holding the measuring apparatus with a hand so that the installation angle of the measuring apparatus varies, the measuring apparatus can measure the viscoelasticity accurately by correcting the zero point.

According to the above-described embodiments, the central position of the operation of the pressing force is displayed in the pressing force display unit; therefore, the measurer can adjust, for example, the inclination of the apparatus so that the central position of the operation of the pressing force becomes appropriate.

According to the above-described embodiments, the pressing force is displayed in a mode of being able to identify the level of the pressing force; therefore, the measurer can adjust, for example, the degree of pressure application of the apparatus so that the pressing force becomes appropriate.

According to the above-described embodiments, the viscoelasticity at the small pressing force is obtained from the viscoelasticity at a plurality of levels of pressing force; therefore, even though the measurement of the measurement target at the small pressing force is difficult, the viscoelasticity at the small pressing force can be obtained.

Modifications

(1) In the embodiments, the control unit is provided in the measuring apparatus main body; however, the present invention is not limited thereto. For example, the measuring apparatus can be connected to a control device such as a personal computer, and a CPU or the like of the control device can be used as the control unit.

(2) In the embodiments, the control unit obtains the displacement of the ball indenter based on the driving pulse of the pulse motor; however, the present invention is not limited thereto. For example, by providing a detection unit such as an optical sensor that detects displacement of the ball indenter, the control unit can be configured to obtain the displacement of the ball indenter based on output from the detection unit.

(3) In the embodiments, the human skin has been exemplified as the measurement target; however, the present invention is not limited thereto. The measuring apparatus according to the embodiments is suitable for soft or thin measurement targets, for example foodstuffs, fibers, rubber, and the like.

(4) In the embodiments, if a noise is generated in output from the load cell (for example, a noise caused by vibration of the load cell accompanying driving of the pulse motor), a filter for cancelling the noise can be provided.

(5) In the embodiments, an example in which the driving unit is disposed directly on the load cell has been presented; however, the present invention is not limited thereto.

For example, the present invention can be configured such that a driving unit fixing part is fixed to a left end part (movable part) side of the load cell and the driving unit is fixed to the driving unit fixing part that is positioned more to the left than the left end part of the load cell.

This configuration has an advantage of reducing steps for processing the existing load cell.

(6) In the embodiments, an example in which the indenter has a ball shape has been presented; however, the present invention is not limited thereto.

**Claims**

1. A viscoelasticity measuring apparatus comprising:

    a casing;
    a surface contact part provided in the casing and brought into surface contact with a measurement target;
    an indenter that moves toward the measurement target over the surface contact part and is pushed into the measurement target;
    a driving unit that supports the indenter and moves the indenter toward the measurement target;
    a load detecting unit of which a fixed part side is fixed to the casing and a movable part side supports the driving unit, wherein the load detecting unit detects a pushing load that pushes the indenter into the measurement target; and
    a displacement obtaining unit that obtains displacement of the indenter.

2. The viscoelasticity measuring apparatus according to claim 1 further comprising a control unit, wherein the control unit performs:

   an indenter pushing process in which the control unit controls the driving unit to position the indenter at a pushing position; and
   a viscoelasticity calculating process in which the control unit calculate viscoelasticity of the measurement target at the pushing position of the indenter pushing process, based on output from the load detecting unit and the displacement of the indenter obtained by the displacement obtaining unit.

3. The viscoelasticity measuring apparatus according to claim 2, wherein:

   the driving unit includes a pulse motor; and
   the control unit controls the pulse motor by outputting a driving pulse, obtains displacement of the indenter based on the driving pulse, and functions as the displacement obtaining unit, wherein:

      the control unit preferably performs the viscoelasticity calculating process by adding displacement of the load detecting unit as displacement of the indenter.

4. The viscoelasticity measuring apparatus according to any of claims 1 to 3, further comprising:

   a contact angle correcting unit that corrects a contact angle of the indenter with respect to a surface of the measurement target to be orthogonal to the surface of the measurement target.

5. The viscoelasticity measuring apparatus according to any of claims 1 to 4, further comprising:

   a contact pressure correcting unit that corrects appropriately pressure of contact between the surface contact part and the measurement target.

6. The viscoelasticity measuring apparatus according to any of claims 1 to 5,
   the viscoelasticity measuring apparatus being used in such a way that a measurer holds the casing and brings the surface contact part into contact with the measurement target, and
   further comprising a vibration reducing unit that reduces vibration between the surface contact part and the measurement target.

7. The viscoelasticity measuring apparatus according to any of claims 1 to 6, further comprising:

   an inclination detecting unit for detecting inclination of a detecting direction of the load detecting unit; and
   a control unit, wherein
   the control unit corrects a detection reference point of the load detecting unit by correcting an amount corresponding to the inclination of the detecting direction detected by the inclination detecting unit with respect to output of load detecting unit.

8. The viscoelasticity measuring apparatus according to Claim 7, wherein:

   the inclination detecting unit detects the detecting direction by detecting acceleration; and
   the control unit corrects the detection reference point of the load detecting unit by offsetting an amount corresponding to the acceleration detected by the inclination detecting unit with respect to the output of the load detecting unit.

9. The viscoelasticity measuring apparatus according to any of claims 1 to 8, further comprising:

   a pressing force detecting unit for detecting pressing force at the surface contact part; and
   a control unit, wherein
   the control unit determines whether the viscoelasticity is measured or not, based on output of the pressing force detecting unit.

10. The viscoelasticity measuring apparatus according to claim 9, wherein
    the control unit measures the viscoelasticity when the control unit has determined output of the pressing force at the surface contact part is within a predetermined pressure on the basis of the pressing force detecting unit.

11. The viscoelasticity measuring apparatus according to claim 9 or 10, further comprising:

a plurality of the pressing force detecting units for detecting the pressing force at a plurality of positions on the surface contact part, wherein
the control unit measures the viscoelasticity when the control unit has determined that a relative value of the pressing force at the plurality of positions is within a predetermined range on the basis of output of the plurality of pressing force detecting units.

12. The viscoelasticity measuring apparatus according to any of claims 9 to 11, further comprising:

an inclination detecting unit for detecting inclination of a detecting direction of the load detecting unit, wherein
the control unit corrects a detection reference point of the pressing force detecting unit on the basis of output of the inclination detecting unit.

13. The viscoelasticity measuring apparatus according to any of claims 9 to 12, further comprising:

a plurality of the pressing force detecting units for detecting the pressing force at a plurality of positions on the surface contact part; and
a pressing force display unit for displaying the pressing force, wherein
the control unit allows the pressing force display unit to display a central position of operation of the pressing force among the plurality of positions on the basis of the output of the pressing force detecting units.

14. The viscoelasticity measuring apparatus according to any of claims 9 to 13, further comprising:

a pressing force display unit for displaying the pressing force, wherein
the control unit allows the pressing force display unit to display the pressing force in a mode of being able to identify a level of the pressing force on the basis of the output of the pressing force detecting unit.

15. The viscoelasticity measuring apparatus according to any of claims 1 to 8, further comprising:

a pressing force detecting unit for detecting pressing force at the surface contact part; and
a control unit, wherein
the control unit calculates the viscoelasticity of the measurement target at a plurality of levels of pressing force with different output of the pressing force detecting unit, and obtains the viscoelasticity corresponding to a lower level of pressing force than the plurality of levels of pressing force on the basis of the calculated plurality of viscoelasticity.

## FIG. 1A

## FIG. 1B

# FIG. 2A

# FIG. 2B

## FIG. 3A

## FIG. 3B

# FIG. 4

MEASURING APPARATUS 1

SUBSTRATE 50

STORAGE UNIT 65

CONTROL UNIT 66

OPERATION UNIT 61

DISPLAY UNIT 62

MOTOR DRIVING CIRCUIT 64

A/D CONVERTER 63

BALL INDENTER 40

DRIVING UNIT 30

PULSE MOTOR 31

LOAD CELL 20

# FIG. 5

START

S1 — HAS OPERATION UNIT BEEN OPERATED? — NO

YES

S2 — SET LOAD TO ZERO

S3 — DRIVE BALL INDENTER IN PUSHING DIRECTION
•START PULSE COUNT (ORIENTATION ACQUISITION)
•START LOAD MEASUREMENT
•START COLLECTING MEASUREMENT DATA OF DISPLACEMENT AND LOAD

S4 — HAS SPECIFIED DISPLACEMENT OR SPECIFIED PUSHING LOAD REACHED? — NO

YES

S5 — •ARRANGE BALL INDENTER AT EVACUATED POSITION
•STOP MEASURING DISPLACEMENT AND LOAD
•STOP COLLECTING MEASUREMENT DATA

S6 — <VISCOELASTICITY CALCULATION PROCESS>
CALCULATE MEASUREMENT RESULT (VISCOELASTICITY) BASED ON COLLECTED DATA OF DISPLACEMENT AND PUSHING LOAD

S7 — DISPLAY MEASUREMENT RESULT

S8 — END

FIG. 6A

FIG. 6B

FIG. 7A

FIG. 7B

FIG. 7C

FIG. 8A

FIG. 8B

SKIN (U1)

SUCTION

FIG. 8C

FIG. 8D

SKIN (U1)

SUCTION

FIG. 9A

301-5

375a  40   11  11a   310   375b                    312

Y2
Y  X
X1 ←→ X2
Y1

375c                              375d

FIG. 9C    11   11a          FIG. 9B      11a        11

θY                                    θX        310           312

310

375a          375c                          375c              375d

312

Z2
Z  Y                                              Z2
Y2 ←→ Y1                                      Z  X
Z1                                          X1 ←→ X2
                                            Z1

FIG. 10A 301-6

40  11a  310  312

Y2
Y
X1 ←→ X2
X
Y1

FIG. 10C

11
11a
θY
376
312

Z2
Z  Y
Y2 ←→ Y1
Z1

FIG. 10B

11a
11
θX
310
312
376

Z2
Z  X
X1 ←→ X2
Z1

EP 2 687 151 A1

# FIG. 11

301-7

# FIG. 12A

401

471-2

40

10

471-1

471-3

Y2
Z2 Y X
X1 ← → X2
Z
Y1 Z1

# FIG. 12B

471-1〜471-3

40

475-1〜475-3

30

480

20

Z2
Z X
X1 ← → X2
Z
Z1

# FIG. 13

MEASURING APPARATUS 401

EP 2 687 151 A1

34

LOAD OF BALL
INDENTER

FIG. 14A

A4

O ——————————————————•———————————————→ DISPLACEMENT

|← A1 →|← A3 →|

A2

LOAD OF BALL
INDENTER

FIG. 14B

B4

O ——————————————————•———————————————→ DISPLACEMENT

|← B1 →|← B3 →|

B2

LOAD OF BALL
INDENTER

FIG. 14C

C4

O ————————————————————————————————————→ DISPLACEMENT

C3

|← C1 →|← C3 →|

C2

# FIG. 15A

OUTPUT OF LOAD CELL
OUTPUT OF ACCELERATION SENSOR

| -90° | -45° | 0° | +45° | +90° |
|------|------|-----|------|------|

# FIG. 15B

OUTPUT OF LOAD CELL

TIME (S)

0
2   4   6   8   10

# FIG. 15C

OUTPUT OF
ACCELERATION SENSOR

TIME (S)

0
2   4   6   8   10

# FIG. 15D

OUTPUT OF LOAD CELL-
OUTPUT OF ACCELERATION SENSOR

TIME (S)

0
2   4   6   8   10

# FIG. 16A

LOAD OF BALL
INDENTER

ZERO POINT IS NOT CORRECTED

DISPLACEMENT

D1

D2

D3

# FIG. 16B

LOAD OF BALL
INDENTER

ZERO POINT IS CORRECTED

DISPLACEMENT

E1

E2

E3

# FIG. 17A

401

F1

U1

F2

471-1～471-3

475-1～475-3

# FIG. 17B

401

F3

U1

F4

471-1～471-3

475-1～475-3

G

# FIG. 18

START

S401 — OPERATION UNIT OPERATED? — NO

YES

S402 — START MEASUREMENT OF INDENTER LOAD, ACCELERATION, AND PRESSING FORCE

S403 — TOTAL PRESSING FORCE AT MULTIPLE POSITIONS IS WITHIN PREDETERMINED RANGE? — NO

YES

S404 — RELATIVE VALUE OF PRESSING FORCE IS WITHIN PREDETERMINED RANGE? — NO

YES

S405 — START DRIVING BALL INDENTER IN PUSHING DIRECTION
·START PULSE COUNT (DISPLACEMENT ACQUISITION)
·START ACCUMULATION OF MEASUREMENT DATA INCLUDING INDENTER LOAD, ACCELERATION, AND PRESSING FORCE

S406 — TOTAL PRESSING FORCE AT MULTIPLE POSITIONS IS WITHIN PREDETERMINED RANGE? — NO

YES

S407 — RELATIVE VALUE OF PRESSING FORCE IS WITHIN PREDETERMINED RANGE? — NO

YES

S407a — CANCEL MEASUREMENT
·RETRACTION DRIVE OF BALL INDENTER

S408 — PREDETERMINED DISPLACEMENT OR PREDETERMINED PUSHING LOAD ACHIEVED?

NO

YES

S409 — DISPOSE BALL INDENTER AT RETRACTION POSITION
·CORRECT INDENTER LOAD USING ACCUMULATED ACCELERATION
·CALCULATE MEASUREMENT RESULT (VISCOELASTICITY) BASED ON CORRECTION DATA

S410 — DISPLAY MEASUREMENT RESULTS

S411 — END

# FIG. 19

UP

493c

RIGHT

LEFT

493b

DOWN

Y2

Y

X

X1

X2

Y

Y1

493a

# FIG. 20

## PRESSING FORCE AND YOUNG'S MODULUS

| PRESSING FORCE | gf | | 0 | 100 | 150 | 200 |
|---|---|---|---|---|---|---|
| | N/m² | | 0 | 2460.075 | 3690.113 | 4920.15 |
| YOUNG'S MODULUS | kPa | FIRST TIME | 13.21 | 20.33 | 22.65 | 26.16 |
| | | SECOND TIME | 12.97 | 20.27 | 23.71 | 29.41 |
| | | THIRD TIME | 13.64 | 17.98 | 22.59 | 24.93 |
| | | AVERAGE | 13.27 | 19.53 | 22.98 | 26.83 |

YOUNG'S MODULUS (kPa)

PRESSING FORCE (N/m²)

EUROPEAN SEARCH REPORT

Europäisches Patentamt
European Patent Office
Office européen des brevets

Application Number

EP 13 00 3670

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/002428 A1 (UNIV SYDNEY [AU]; FIELD JOHN SUGDEN [AU]; SWAIN MICHAEL VINCENT [AU];) 13 January 2005 (2005-01-13) * abstract * * page 2, line 20 - page 7, line 2; figures 4,5 * | 1-15 | INV. A61B5/00 |
| X | JP 2009 240374 A (UNIV OSAKA; WAVE CYBER KK) 22 October 2009 (2009-10-22) * abstract * * An automated computer translation of this Japanese publication can be found on the website of the Japanese Patent Office: http://ipdl.inpit.go.jp/homepg e.ipdl; figures 2,3 * | 1-15 | |
| X | WO 2010/082356 A1 (M I LAB [JP]; TAKASHIMA MITSURU [JP]) 22 July 2010 (2010-07-22) * abstract; figures 1-5 * * An automated computer translation of this Japanese publication can be found on the website of the Japanese Patent Office: http://ipdl.inpit.go.jp/homepg e.ipdl * | 1-15 | TECHNICAL FIELDS SEARCHED (IPC) A61B |
| A,D | JP 2011 137667 A (UNIV TOKYO AGRICULTURE) 14 July 2011 (2011-07-14) * abstract; figures 1,5 * * An automated computer translation of this Japanese publication can be found on the website of the Japanese Patent Office: http://ipdl.inpit.go.jp/homepg e.ipdl * | 1-15 | |

The present search report has been drawn up for all claims

| Place of search | Date of completion of the search | Examiner |
|---|---|---|
| The Hague | 17 September 2013 | Juárez Colera, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 13 00 3670

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2013

| Patent document cited in search report | | Publication date | Patent family member(s) | | Publication date |
|---|---|---|---|---|---|
| WO 2005002428 | A1 | 13-01-2005 | NONE | | |
| JP 2009240374 | A | 22-10-2009 | NONE | | |
| WO 2010082356 | A1 | 22-07-2010 | NONE | | |
| JP 2011137667 | A | 14-07-2011 | JP<br>JP | 5076253 B2<br>2011137667 A | 21-11-2012<br>14-07-2011 |

For more details about this annex : see Official Journal of the European Patent Office, No. 12/82

**REFERENCES CITED IN THE DESCRIPTION**

**Patent documents cited in the description**

- JP 2012161478 A
- JP 2013147258 A
- JP 2000316818 A
- JP 2011137667 A
- WO 2010084840 A